# EUROPEAN PATENT APPLICATION

(11) **EP 4 289 863 A1**
(43) Date of publication of application: **13.12.2023**
(21) Application number: 21905768.4
(22) Date of filing: 16.12.2021
(51) Int. Cl.: C07K 16/28, A61P 17/06, A61K 39/395

(54) **BISPECIFIC ANTIBODY TARGETING IL-17A AND IL-36R AND APPLICATION THEREOF**

(30) Priority: 17.12.2020 CN 202011502873
(71) Applicant: Shanghai Huaota Biopharmaceutical Co., Ltd., Shanghai 201203 (CN); Huabo Biopharm (Shanghai) Co., Ltd., Shanghai 201203 (CN); ZHEJIANG HUAHAI BIOPHARMACEUTICALS CO., LTD., Hangzhou, Zhejiang 310018 (CN)
(72) Inventor: XU, Jingen, Shanghai 201203 (CN); ZHU, Xiangyang, Shanghai 201203 (CN); MA, Xiaojuan, Shanghai 201203 (CN); YU, Haijia, Shanghai 201203 (CN); JIA, Huifeng, Shanghai 201203 (CN); WANG, Yan, Shanghai 201203 (CN); CUI, Xiaopei, Shanghai 201203 (CN)
(74) Representative: Weickmann & Weickmann PartmbB
(86) International application number: PCT/CN2021/138634
(87) International publication number: WO 2022/127842

(57) **Abstract**

Provided are a bispecific antibody targeting IL-17A and IL-36R and an application thereof. Particularly, provided is a bispecific antibody targeting IL-17A and IL-36R and having high affinity and high biological activity. The antibody can implement high-affinity binding to IL-36R and IL-17A, and can block the binding of IL-36R ligands (α, β, γ) to IL-36R and the binding of IL-17A to IL-17R, thereby treating and/or preventing IL-36 and/or IL-17 related diseases.

## Description

### FIELD OF THE INTVENTION

The present application relates to the field of biomedicine, and in particular, relates to a bispecific antibody targeting IL-17A and IL-36R and an application thereof.

### BACKGROUND OF THE INVENTION

The IL-17 family contains six members, including IL-17A (IL-17), IL-17B, IL-17C, IL-17D, IL-17E (IL-25), and IL-17F. These interleukin-17 cytokines can bind to corresponding receptors and thus mediate different inflammatory responses. IL-17A is a homodimer formed by linking two chains of 155 amino acids via a disulfide bond, with a molecular weight of 35 kDa. Structurally, the IL-17 consists of a signal peptide (AA) composed of 23 amino acids, and a region of a 123-amino-acid chain. IL-17 binds to receptors, called IL-17R, on the surfaces of type I cells, and the receptors have at least three forms, namely: IL-17RA, IL-17RB, and IL-17RC. IL-17A and IL-17F bind to IL-17RA and IL-17RC receptor complexes in the form of homodimers or heterodimers to transduce signals, and participate in autoimmune diseases, multiple inflammatory responses, and host anti-infective immune responses in an organism. IL-17C binds to IL-17RA and IL-17RE receptor complexes to activate downstream signals and promote anti-infective immunity, autoimmune diseases and inflammatory responses in an organism.

IL-36 is a new member in the IL-1 family and includes IL-36α, IL-36β, IL-36γ, and an IL-36 receptor antagonist (IL-36Ra). An IL-36 receptor (IL-36R) is a heterodimer consisting of an IL-1 receptor-related protein 2 (IL-1Rrp2) and a co-receptor IL-1 receptor accessory protein (IL-1RAc P). IL-36α, IL-36β and IL-36γ can promote inflammatory cytokine expression by binding to IL-36R to activate mitogen-activated protein kinases and nuclear factor-κB signaling pathway, while IL-36Ra can competitively bind to IL-36R to block its signal transduction and thus inhibit inflammatory cytokine expression. In a human body, dysregulation of a gene encoding IL-36Ra can lead to the development of disseminated pustular psoriasis.

Recent studies have shown that an IL-17 signaling pathway can work synergistically with an IL-36R signaling pathway to regulate the anti-infective immunity, autoimmune diseases and inflammatory responses in an organism. The preparation of a bispecific antibody targeting these two targets simultaneously has the potential to produce better therapeutic effects on autoimmune diseases such as psoriasis. Therefore, it is necessary to develop a new bispecific antibody targeting IL-17A and IL-36R with strong specificity and high affinity in the art.

### SUMMARY OF THE INVENTION

An object of the present application is to provide a bispecific antibody targeting IL-17A and IL-36R and an application thereof.

Another object of the present application is to provide an IL-36R antibody that can be used to construct a bispecific antibody targeting IL-17A and IL-36R with high affinity and high bioactivity. The IL-36R is capable of binding to IL-36R with high affinity and blocking the binding of IL-36α, IL-36β, or IL-36γ to IL-36R.

Another object of the present application is to provide an interleukin-36R binding molecule and uses thereof, in particular uses in the treatment and/or prevention, or diagnosis of IL-36-related diseases such as psoriasis.

Another object of the present application is to provide a polyspecific antibody having one or more of the following properties: (1) capability of specifically binding to human IL-36R and human IL-17A with high affinity, respectively; (2) capability of simultaneously binding to an IL-36R protein and an IL-17A protein; (3) capability of keeping a binding configuration of the polyspecific antibody with a bound target unchanged and maintaining molecular stability; (4) the purity of above 90% for the polyspecific antibody under a preliminary culture condition; (5) capability of maintaining the binding affinity of the bound target, and exerting the synergistic effect of dual targets as compared a single anti-IL-36R antibody and a single anti-IL-17A antibody; and (6) capability of effectively regulating anti-infective immunity, autoimmune diseases and inflammatory responses, for example, psoriasis, in an organism. A method for preparing the polyspecific antibody of the present application is simple and feasible, and has a good application prospect.

The first aspect of the present application provides a heavy-chain variable region of an antibody. The heavy-chain variable region comprises the following three complementary determining regions (CDRs):
CDR1 (GYTFTSSW) as set forth in SEQ ID NO: 3,
CDR2 (IHPNSAKT) as set forth in SEQ ID NO: 4, and
CDR3 (ARVDYGKPWFAY) as set forth in SEQ ID NO: 5.

In some embodiments, any of the above amino acid sequences further comprises a derivative sequence, which has at least one (such as 1 to 3, for example, possibly 1 to 2, for example, possibly 1) amino acid optionally added, deleted, modified and/or substituted and can retain the IL-36R binding affinity.

In some embodiments, the heavy-chain variable region further comprises a human-derived FR or a murine FR.

In some embodiments, the heavy-chain variable region has an amino acid sequence as set forth in SEQ ID NO: 1.

In some embodiments, the heavy-chain variable region has an amino acid sequence as set forth in SEQ ID NO: 9.

The second aspect of the present application provides an antibody heavy chain, comprising the heavy-chain variable region as described in the first aspect of the present application.

In some embodiments, the antibody heavy chain further comprises a heavy-chain constant region.

In some embodiments, the heavy-chain constant region is human-derived, murine, or rabbit-derived.

The third aspect of the present application provides a light-chain variable region of an antibody. The light-chain variable region comprises the following three complementary determining regions (CDRs):
CDR1' (SSVSSSY) as set forth in SEQ ID NO: 6,
CDR2' (STS) as set forth in SEQ ID NO: 7, and
CDR3' (QQFQSSPLT) as set forth in SEQ ID NO: 8.

In some embodiments, any of the above amino acid sequences further comprises a derivative sequence, which has at least one (such as 1 to 3, for example, possibly 1 to 2, for example, possibly 1) amino acid optionally added, deleted, modified and/or substituted and can retain the IL-36R binding affinity.

In some embodiments, the light-chain variable region further comprises a human-derived FR or a murine FR.

In some embodiments, the light-chain variable region has an amino acid sequence as set forth in SEQ ID NO: 2.

In some embodiments, the light-chain variable region has an amino acid sequence as set forth in SEQ ID NO: 10, 11, or 12.

The fourth aspect of the present application provides an antibody light chain, comprising the light-chain variable region as described in the third aspect of the present application.

In some embodiments, the antibody light chain further comprises a light-chain constant region.

In some embodiments, the light-chain constant region is human-derived, murine, or rabbit-derived.

The fifth aspect of the present application provides an anti-IL-36R antibody, comprising:
(1) the heavy-chain variable region as described in the first aspect of the present application; and/or
(2) the light-chain variable region as described in the third aspect of the present application;
or, the antibody has: the heavy chain as described in the second aspect of the present application; and/or the light chain as described in the fourth aspect of the present application.

In some embodiments, an affinity constant KD (M) of the antibody for binding to a human IL-36R protein (for example, which may be a wild type) is (0.5-10)×10⁻¹¹, for example, which may be (1-6)×10⁻¹¹, for example, which may be (1-2)×10⁻¹¹.

In some embodiments, an affinity constant KD(M) of the antibody for binding to human IL-1 Rrp2 is (0.5-10) × 10⁻¹¹, for example, which may be (1-6)×10⁻¹¹, for example, which may be (1-2)×10⁻¹¹.

In some embodiments, the antibody is capable of blocking the binding of IL-36α, IL-36β, or IL-36γ to IL-36R.

In some embodiments, the antibody is capable of blocking cytokine secretion mediated by IL-36α, IL-36β, or IL-36γ. For example, the cytokine may comprise: IL-6, IL-8, and GM-CSF.

In some embodiments, the antibody is selected from: an animal-derived antibody, a chimeric antibody, a humanized antibody, or combinations thereof.

In some embodiments, the antibody is a double-chain antibody or a single-chain antibody.

In some embodiments, the antibody is a monoclonal antibody.

In some embodiments, the antibody is a partially or fully humanized monoclonal antibody.

In some embodiments, the heavy-chain variable region of the antibody has a sequence as set forth in SEQ ID NO: 1 or 9; and/or
the light-chain variable region of the antibody has a sequence as set forth in SEQ ID NO: 2, 10, 11 or 12.

In some embodiments, the heavy-chain variable region of the antibody has a sequence as set forth in SEQ ID NO: 1; and the light-chain variable region of the antibody has a sequence as set forth in SEQ ID NO: 2.

In some embodiments, the heavy-chain variable region of the antibody has a sequence as set forth in SEQ ID NO: 9; and the light-chain variable region of the antibody has a sequence as set forth in SEQ ID NO: 10, 11 or 12.

In some embodiments, the antibody is in the form of a drug conjugate.

In the sixth aspect of the present application, the present application provides a polyspecific antibody, comprising a first targeting moiety capable of specifically binding to an IL-36R protein, wherein the first targeting moiety comprises an isolated antigen-binding protein comprising a heavy-chain variable region (VH), which comprises the following three complementarity determining regions (CDRs):
CDR1 as set forth in SEQ ID NO: 3,
CDR2 as set forth in SEQ ID NO: 4, and
CDR3 as set forth in SEQ ID NO: 5.

In some embodiments, any of the above amino acid sequences further comprises a derivative sequence, which has at least one (such as 1 to 3, for example, possibly 1 to 2, for example, possibly 1) amino acid optionally added, deleted, modified and/or substituted and can retain the IL-36R binding affinity.

In some embodiments, the heavy-chain variable region further comprises a human-derived FR or a murine FR.

In some embodiments, the heavy-chain variable region has an amino acid sequence as set forth in SEQ ID NO: 1.

In some embodiments, the heavy-chain variable region has an amino acid sequence as set forth in SEQ ID NO: 9.

In another aspect of the present application, the polyspecific antibody comprises a light-chain variable region (VL), which comprises the following three complementary determining regions (CDRs):
CDR1' as set forth in SEQ ID NO: 6,
CDR2' as set forth in SEQ ID NO: 7, and
CDR3' as set forth in SEQ ID NO: 8.

In some embodiments, any of the above amino acid sequences further comprises a derivative sequence, which has at least one (such as 1 to 3, for example, possibly 1 to 2, for example, possibly 1) amino acid optionally added, deleted, modified and/or substituted and can retain the IL-36R binding affinity.

In some embodiments, the light-chain variable region further comprises a human-derived FR or a murine FR.

In some embodiments, the light-chain variable region has an amino acid sequence as set forth in SEQ ID NO: 2.

In some embodiments, the light-chain variable region has an amino acid sequence as set forth in SEQ ID NO: 10, 11 or 12.

In some embodiments, the polyspecific antibody further comprises a second targeting moiety, for example, which may specifically bind to IL-17A.

The seventh aspect of the present application provides a bispecific antibody, comprising:
(a) an anti-IL-17A antibody; and
(b) a single-chain variable region (ScFv) of an anti-IL-36R antibody linked to the anti-IL-17A antibody.

In some embodiments, the anti-IL-17A antibody and the ScFv of the anti-IL-36R antibody are linked via a linker sequence.

In some embodiments, the ScFv of the anti-IL-36R antibody is linked to a region of the anti-IL-17A selected from regions in the group consisting of: a heavy-chain variable region, a heavy-chain constant region, or combinations thereof.

In some embodiments, the ScFv of the anti-IL-36R antibody is linked to a terminus of the heavy-chain constant region of the anti-IL-17A antibody.

In some embodiments, the anti-IL-17A antibody is humanized.

In some embodiments, the ScFv of the IL-36R antibody is humanized.

In some embodiments, the bispecific antibody is a homodimer.

In some embodiments, the bispecific antibody is a tetravalent antibody.

In some embodiments, the bispecific antibody has a structure from an N-terminus to a C-terminus as set forth in Formula I: 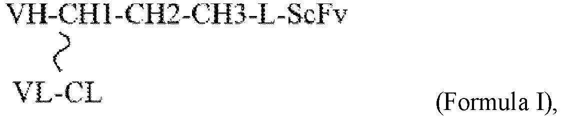 wherein,
VH represents the heavy-chain variable region of the anti-IL-17A antibody;
VL represents the light-chain variable region of the anti-IL-17A antibody;
CHI, CH2 and CH3 represent the heavy-chain constant regions CH1, CH2 and CH3 of the anti-IL-17A antibody, respectively;
CL represents the light-chain constant region of the anti-IL-17A antibody;
ScFv represents the ScFv of the anti-IL-36R antibody;
L indicates a linker element;
"~" represents a disulfide bond;
"-" represents a peptide bond; and
wherein the bispecific antibody has an activity of simultaneously binding to the IL-17A and the IL-36R.

In some embodiments, the bispecific antibody is fused from the anti-IL-17A antibody and the ScFv of the anti-IL-36R antibody, and has two pairs of peptide chains symmetrical to each other, each pair of the peptide chains comprises a light chain L-chain and a heavy chain H-chain, and all the peptide chains are linked by the disulfide bonds, wherein any pair of the peptide chains have the structures of the H-chain and the L-chain from the N-terminus to the C-terminus as set forth in Formula I.

In some embodiments, the bispecific antibody is a homodimer of the peptide chains having the structures as set forth in Formula I.

In some embodiments, the linker element is a linker peptide, for example, which may have an amino acid sequence of (G4S)n, wherein n is a positive integer (for example, 1, 2, 3, 4, 5 or 6), for example, which may be 4.

In some embodiments, the ScFv of the anti-IL-36R antibody comprises an anti-IL-36R heavy-chain variable region and an anti-IL-36R light-chain variable region.

In some embodiments, the ScFv of the anti-IL-36R antibody further comprises a linker peptide located between the heavy-chain variable region of the anti-IL-36R antibody and the light-chain variable region of the anti-IL-36R antibody for linking the heavy-chain variable region to the light-chain variable region.

In some embodiments, the linker element has an amino acid sequence of (G4S)n, wherein n is a positive integer (for example, 1, 2, 3, 4, 5 or 6), for example, which may be 4.

In some embodiments, the heavy-chain variable region of the anti-IL-17A antibody comprises the following three complementary determining regions (CDRs):
CDR1 as set forth in SEQ ID NO: 20,
CDR2 as set forth in SEQ ID NO: 21, and
CDR3 as set forth in SEQ ID NO: 22.

In some embodiments, the light-chain variable region of the anti-IL-17A antibody comprises the following three complementary determining regions (CDRs):
CDR1' as set forth in SEQ ID NO: 23,
CDR2' as set forth in SEQ ID NO: 24, and
CDR3' as set forth in SEQ ID NO: 25.

In some embodiments, in the ScFv of the anti-IL-17A antibody, the heavy-chain variable region is as set forth in SEQ ID NO: 26.

In some embodiments, in the ScFv of the anti-IL-17A antibody, the light-chain variable region is as set forth in SEQ ID NO: 27.

In some embodiments, the heavy-chain variable region of the anti-IL-36R antibody comprises the following three complementary determining regions (CDRs):
CDR1 as set forth in SEQ ID NO: 3,
CDR2 as set forth in SEQ ID NO: 4, and
CDR3 as set forth in SEQ ID NO: 5.

In some embodiments, the light-chain variable region of the anti-IL-36R antibody comprises the following three complementary determining regions (CDRs):
CDR1' as set forth in SEQ ID NO: 6,
CDR2' as set forth in SEQ ID NO: 7, and
CDR3' as set forth in SEQ ID NO: 8.

In some embodiments, in the ScFv of the anti-IL-36R antibody, the heavy-chain variable region of the anti-IL-36R antibody has an amino acid sequence as set forth in SEQ ID NO: 1, or with ≥85% (for example, which may be 90%, for example, 95%) sequence identity to the sequence as set forth in SEQ ID NO: 1; and the light-chain variable region of the anti-IL-36R antibody has an amino acid sequence as set forth in SEQ ID NO: 2, or with ≥85% (for example, which may be 90%, for example, 95%) sequence identity to the sequence as set forth in SEQ ID NO: 2.

In some embodiments, in the ScFv of the anti-IL-36R antibody, the heavy-chain variable region of the anti-IL-36R antibody has an amino acid sequence as set forth in SEQ ID NO: 9, or with ≥85% (for example, which may be 90%, for example, 95%) sequence identity to the sequence as set forth in SEQ ID NO: 9; and the light-chain variable region of the anti-IL-36R antibody has an amino acid sequence as set forth in SEQ ID NO: 10, 11 or 12, or with ≥85% (for example, which may be 90%, for example, 95%) sequence identity to the sequence as set forth in SEQ ID NO: 10, 11 or 12.

In some embodiments, in the ScFv of the anti-IL-36R antibody, the heavy-chain variable region has an amino acid sequence as set forth in SEQ ID NO: 9; and the light-chain variable region has an amino acid sequence as set forth in SEQ ID NO: 11.

In some embodiments, in the ScFv of the anti-IL-36R antibody, an amino acid residue G at position 44 is mutated into C in the amino acid sequence of the heavy-chain variable region of the anti-IL-36R antibody, based on SEQ ID NO: 9.

In some embodiments, in the ScFv of the anti-IL-36R antibody, an amino acid residue Q at position 101 is mutated into C in the amino acid sequence of the light-chain variable region of the anti-IL-36R antibody, based on SEQ ID NO: 11.

In some embodiments, the heavy-chain constant regions CH1, CH2 and CH3 and light-chain constant region CL of the anti-IL-17A antibody are both derived from human IgG1 or IgG4, which can be, for example, human IgG4.

In some embodiments, the H-chain of the bispecific antibody has an amino acid sequence as set forth in anyone of SEQ ID NO: 13 to 18; and the L-chain of the bispecific antibody has an amino acid sequence as set forth in SEQ ID NO: 19.

In some embodiments, the bispecific antibody further comprises (for example, may be conjugated with) a detectable label, a targeted marker, a drug, a toxin, a cytokine, a radionuclide, or an enzyme.

In some embodiments, the bispecific antibody is conjugated with a tumor-targeted marker conjugate.

In some embodiments, the bispecific antibody of the present application further comprises an active fragment and/or derivative of the antibody, wherein the derivative comprises the active fragment of the bispecific antibody, and/or the derivative retains 70%, 75%, 80%, 85%, 90%, 95%, 96%, 97%, 98%, 99%, 100% of the anti-IL-17A and/or anti-IL-36R activity.

The sixth aspect of the present application provides a recombinant protein, comprising:
(i) the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application; and
(ii) optionally a tag sequence assisting expression and/or purification.

In some embodiments, the tag sequence comprises a 6His tag.

In some embodiments, the recombinant protein (or polypeptide) comprises a fusion protein.

In some embodiments, the recombinant protein is a monomer, a dimer, or a multimer.

The eighth aspect of the present application provides a CAR construct, wherein an scFV segment of an antigen-binding region of a monoclonal antibody of the CAR construct is a binding region specifically binding to IL-36R, and the scFv has the heavy-chain variable region as described in the first aspect of the present application and the light-chain variable region as described in the third aspect of the present application.

The ninth aspect of the present application provides a recombinant immune cell expressing the exogenous CAR construct as described in the seventh aspect of the present application.

In some embodiments, the immune cell is selected from the group consisting of: NK cells and T cells.

In some embodiments, the immune cell is derived from human or a non-human mammal (such as mouse).

The tenth aspect of the present application provides an antibody-drug conjugate, comprising:
(a) an antibody moiety selected from the group consisting of: the heavy-chain variable region as described in the first aspect of the present application, the heavy chain as described in the second aspect of the present application, the light-chain variable region as described in the third aspect of the present application, the light chain as described in the fourth aspect of the present application, the antibody as described in the fifth aspect of the present application, the polyspecific antibody as described in the sixth aspect of the present application, the bispecific antibody as described in the seventh aspect of the present application, or combinations thereof; and
(b) a conjugated moiety conjugated to the antibody moiety, wherein the conjugated moiety is selected from the group consisting of: a detectable label, a drug, a toxin, a cytokine, a radionuclide, an enzyme, or combinations thereof.

In some embodiments, the conjugated moiety is selected in part from the group consisting of: a fluorescent or luminescent label, a radioactive label, an MRI (magnetic resonance imaging) or CT (electronic computed tomography) contrast agent, an enzyme capable of producing a detectable product, a radionuclide, a biotoxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (for example, DT-diaphorase (DTD) or a biphenylhydrolase-like protein (BPHL), a chemotherapeutic agent (for example, cisplatin) or any form of nanoparticles, or other active substances.

In some embodiments, the antibody moiety is conjugated to the conjugated moiety by a chemical bond or linker.

The eleventh aspect of the present application provides use of an active ingredient selected from the group consisting of: the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application, the immune cell as described in the ninth aspect of the present application, the antibody-drug conjugate as described in the tenth aspect of the present application, or combinations thereof. The active ingredient is for use in (a) preparation of a detection reagent or kit; and/or (b) preparation of a drug for prevention and/or treatment of an IL-36-related disease.

Another aspect of the present application provides use of the polyspecific antibody as described in the sixth aspect of the present application or the bispecific antibody as described in the seventh aspect of the present application in (a) preparation of a detection reagent or kit against IL-36 and/or IL-17; and/or (b) preparation of a drug for prevention and/or treatment of an IL-36- and/or IL-17-related disease.

In some embodiments, the IL-36- and/or IL-17-related disease is an IL-36- and/or IL-17-mediated inflammatory disease.

In some embodiments, the IL-36- and/or IL-17-related disease is a disease induced by overstimulation or mutation of an IL-36 and/or IL-17 cytokine.

In some embodiments, the IL-36- and/or IL-17-related disease is selected from the group consisting of: inflammation, autoimmune diseases, or a combination thereof. For example, the IL-36- and/or IL-17-related disease may be an autoimmune disease.

In some embodiments, the IL-36- and/or IL-17-related disease is selected from the group consisting of: psoriasis, scleroderma, a chronic kidney disease, an inflammatory bowel disease, psoriatic arthritis, ankylosing spondylitis, multiple sclerosis, inflammatory arthritis, asthma, allergy, or combinations thereof. For example, the IL-36- and/or IL-17-related disease may be psoriasis.

In some embodiments, the psoriasis comprises psoriasis vulgaris, erythrodermic psoriasis, arthritic psoriasis, generalized pustular psoriasis (GPP), and palmoplantar pustulosis (PPP).

In some embodiments, the drug is for use in blocking of binding of IL-36α, IL-36β, or IL-36γ to IL-36R.

In some embodiments, the drug is also for use in blocking of an interaction between IL-17A and IL-17R.

In some embodiments, the drug is for use in blocking of cytokine secretion medicated by IL-36α, IL-36β, or IL-36γ.

In some embodiments, the drug is also for use in blocking of cytokine secretion medicated by IL-17A.

In some embodiments, the antibody is in the form of a drug conjugate (ADC).

In some embodiments, the detection reagent or kit is for use in diagnosis of an IL-36- and/or IL-17-related disease.

In some embodiments, the detection reagent or kit is for use in detection of an IL-36R and/or IL-17A protein in a sample.

In some embodiments, the detection reagent is a detection tablet.

The twelfth aspect of the present application provides a pharmaceutical composition, comprising:
(i) an active ingredient selected from the group consisting of: the heavy-chain variable region as described in the first aspect of the present application, the heavy chain as described in the second aspect of the present application, the light-chain variable region as described in the third aspect of the present application, the light chain as described in the fourth aspect of the present application, or the antibody as described in the fifth aspect of the present application, the polyspecific antibody as described in the sixth aspect of the present application or the bispecific antibody as described in the seventh aspect of the present invention, the immune cell as described in the ninth aspect of the present application, the antibody-drug conjugate as described in the tenth aspect of the present application, or combinations thereof; and
(ii) a pharmaceutically acceptable carrier.

In some embodiments, the pharmaceutical composition is a liquid preparation.

In some embodiments, the pharmaceutical composition is an injection.

The thirteenth aspect of the present application provides a polynucleotide encoding a polypeptide selected from the group consisting of:
(1) the heavy-chain variable region as described in the first aspect of the present application, the heavy chain as described in the second aspect of the present application, the light-chain variable region as described in the third aspect of the present application, the light chain as described in the fourth aspect of the present application, the antibody as described in the fifth aspect of the present application, the polyspecific antibody as described in the sixth aspect of the present application or the bispecific antibody as described in the seventh aspect of the present application; or
(2) the recombinant protein as described in the present application;
(3) the CAR construct as described in the eighth aspect of the present application.

The fourteenth aspect of the present application provides a vector comprising the polynucleotide as described in the thirteenth aspect of the present application.

In some embodiments, the vector comprises: a bacterial plasmid, a bacteriophage, a yeast plasmid, a plant cell virus, a mammalian cellular virus such as an adenovirus, a retrovirus, or other vectors.

The fifteenth aspect of the present invention provides a genetically engineered host cell, comprising the vector as described in the fourteenth aspect of the present application or the polynucleotide as described in the thirteenth aspect of the present application, incorporated in a genome.

The sixteenth aspect of the present application provides a method for *in vitro* detection (including diagnostic or non-diagnostic detection) of an IL-36R and/or IL-17A protein in a sample, comprising the steps of:
(1) contacting, *in vitro,* the sample with the antibody as described in the fifth aspect of the present application, or the polyspecific antibody as described in the sixth aspect of the present application, or the bispecific antibody as described in the seventh aspect of the present application or the antibody-drug conjugate as described in the tenth aspect of the present application; and
(2) detecting whether an antigen-antibody complex is formed, wherein the formation of the complex indicates the presence of the IL-36R protein in the sample.

In some embodiments, the method is for non-diagnostic treatment.

The seventeenth aspect of the present application provides a detection plate, comprising: a substrate (support plate) and a detection strip, wherein the detection strip comprises the antibody as described in the fifth aspect of the present application, the polyspecific antibody as described in the sixth aspect of the present application, or the bispecific antibody as described in the seventh aspect of the present application or the antibody-drug conjugate as described in the tenth aspect of the present application.

The eighteenth aspect of the present application provides a kit, comprising:
(1) a first container comprising the antibody as described in the fifth aspect of the present application, or the polyspecific antibody as described in the sixth aspect of the present application, or the bispecific antibody as described in the seventh aspect of the present application; and/or
(2) a second container comprising an antibody against the antibody as described in the fifth aspect of the present application, or the polyspecific antibody as described in the sixth aspect of the present application, or the bispecific antibody as described in the seventh aspect of the present application;
or, the kit comprises the detection plate as described in the seventeenth aspect of the present application.

The nineteenth aspect of the present application provides a method for preparing a recombinant polypeptide, comprising the steps of:
(a) culturing the host cell as described in the fifteenth aspect of the present application under conditions suitable for expression; and
(b) isolating a recombinant polypeptide from a culture, the recombinant polypeptide being the antibody as described in the fifth aspect of the present application or the polyspecific antibody as described in the sixth aspect of the present application, or the bispecific antibody as described in the seventh aspect of the present application.

The twentieth aspect of the present application provides a method for preventing/or treating an IL-36- and/or IL-17A-related disease, comprising: administering the antibody as described in the fifth aspect of the present application, the polyspecific antibody as described in the sixth aspect of the present application, the bispecific antibody as described in the seventh aspect of the present application, the antibody-drug conjugate of the antibody, or a CAR-T cell expressing the antibody a combinations thereof, to a subject in need thereof.

In some embodiments, the method further comprises: administering other drugs or therapies to the subject in need thereof for combined treatment.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the invention involved in the present application are listed in the appended claims. The characteristics and advantages of the invention involved in the present application may be better understood by referring to the exemplary embodiments and the accompanying drawings described in detail below. A brief description of the drawings is as follows:
FIG. 1 shows the results of cytokine IL-6 release in a bioactivity assay for humanized candidate antibodies;
FIG. 2 shows the results of cytokine IL-8 release in a bioactivity assay for humanized candidate antibodies;
FIG. 3 shows the results of cytokine GM-CSF release in a bioactivity assay for humanized candidate antibodies;
FIG. 4 shows the results of NF-κB phosphorylation in a bioactivity assay for humanized candidate antibodies;
FIG. 5 shows the cell binding activity of candidate antibodies to human IL-36R;
FIG. 6 shows the cell binding activity of candidate antibodies to monkey IL-36R;
FIG. 7 shows the functional inhibition effects of candidate antibodies against huIL-36 stimulating factors in NCI/ADR-RES cells;
FIG. 8 shows the functional inhibition effects of candidate antibodies against huIL-36 stimulating factors in HIF cells;

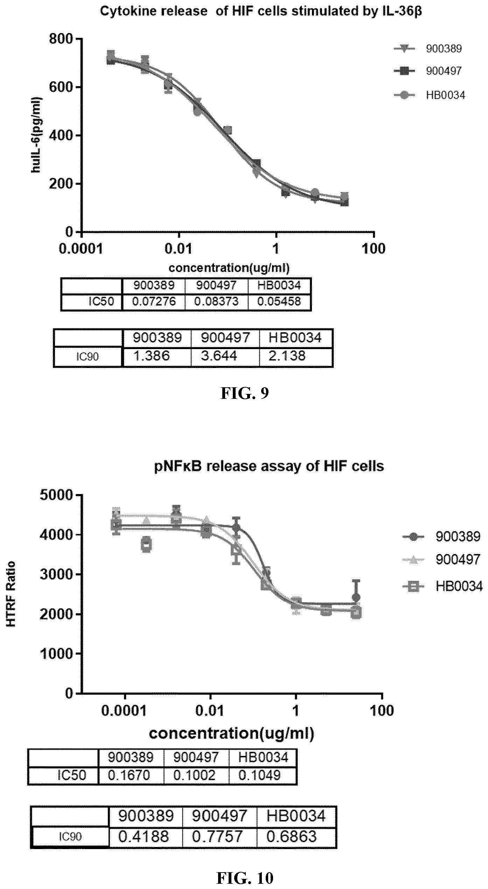

FIG. 9 shows the functional inhibition effects of candidate antibodies against huIL-36 stimulating factors in HIF cells;
FIG. 10 shows the functional inhibition effects of candidate antibodies against huIL-36 stimulating factors in HIF cells;
FIG. 11 shows the molecular structure of an IL17A-IL36R bispecific antibody;
FIG. 12 shows reduced and non-reduced SDS-PAGE patterns of IL17A-IL36R bispecific antibodies;
FIG. 13 shows the results of molecular sieve analysis after one-step affinity chromatography of an IL17A-IL36R bispecific antibody 700009;
FIG. 14 shows the affinity of the IL17A-IL36R bispecific antibody to cell surface IL-36R;
FIG. 15 shows the affinity of an IL17A-IL36R bispecific antibody 700005 to an IL-17A soluble recombinant protein;
FIG. 16 shows the functional inhibition effects of the IL17A-IL36R bispecific antibodies against huIL-36 stimulating factors in NCI/ADR-RES cells;
FIG. 17 shows the functional inhibition effects of the IL17A-IL36R bispecific antibodies against huIL-17A stimulating factors in HT-1080 cells; and
FIG. 18 shows the results of the IL17A-IL36R bispecific antibody blocking IL-6 secretion by IL-36α-activated moDCs.

### DETAILED DESCRIPTION OF THE PREFERRED EMBODIMENTS

Based on extensive and in-depth researches, the applicants have unexpectedly obtained an IL-36R antibody with high affinity and high bioactivity. Experiments show that the IL-36R antibody of the present application can bind to IL-36R with high affinity, and block the binding of IL-36R ligands (α, β, γ) to IL-36R to block the signaling pathway activated by the IL-36R ligands, thereby treating and/or preventing IL-36-related diseases. The present invention is implemented on this basis.

### Terms

As used herein, the terms "administration" and "treatment" refer to the application of exogenous medicaments, therapeutic agents, diagnostic agents, or compositions to animals, humans, subjects, cells, tissues, organs, or biological fluids. "Administration" and "treatment" may refer to therapies, pharmacokinetics, diagnosis, studies, and experimental methods. The treatment of cells comprises contacting a reagent with a cell, contacting a reagent with a fluid, and contacting a fluid with a cell. "Administration" and "treatment" also mean *in vitro* and *ex vivo* treatment by means of reagents, diagnostics, and binding compositions, or by another cell. When applied to humans, animals, or research subjects, "treatment" refers to therapeutic treatment, prevention or preventive measures, study, and diagnosis. It encompasses contacting IL-36R antibodies with humans or animals, subjects, cells, tissues, physiological compartments, or physiological fluids.

As used herein, the term "therapy" refers to the administration of an internally or externally applied therapeutic agent, which comprises any of the IL-36R antibodies and compositions thereof in the present application, to a patient having one or more disease symptoms, and furthermore, the therapeutic agent is known to show a therapeutic effect against these symptoms. The therapeutic agent is usually administered to the patient at an amount (therapeutically effective amount) for effectively alleviating one or more disease symptoms.

As used herein, the term "optional" or "optionally" means that the event or situation described subsequently may occur but does not have to occur. For example, "optionally comprising 1 to 3 antibody heavy-chain variable regions" refers to that a particular sequence may have but not necessarily have 1, 2 or 3 antibody heavy-chain variable regions.

### Antibody

As used herein, the term "antibody" refers to immunoglobulin, which is a tetrapeptide chain structure connected by interchain disulfide bonds between two identical heavy chains and two identical light chains. Different immunoglobulin heavy chain constant regions exhibit different amino acid compositions and rank orders, thereby presenting different antigenicity. Accordingly, immunoglobulins can be divided into five categories, or called immunoglobulin isotypes, namely IgM, IgD, IgG; IgA and IgE, which correspond to heavy-chain constant regions, called α, ε, δ, γ and µ, of different types of immunoglobulins. IgG represents one of the most important classes of immunoglobulins, and due to differences in its chemical structure and biological function, it can be divided into four sub-categories: IgG1, IgG2, IgG3, and IgG4. Light chains are divided into κ or λ chains considering of different constant regions. The subunit structures and three-dimensional configurations of immunoglobulins in different categories are well known to those in the art.

Near the N-terminal of the antibody heavy chains and light chains, the sequence of about 110 amino acids varies largely, known as the variable region (V region); and the rest of the amino acid sequence near the C-terminal is relative stable, known as the constant region (C region). The variable region comprises three hypervariable regions (HVR) and four FRs with a relatively conserved sequence. The amino acid sequence of the four FRs are relatively conserved and are not directly involved in a binding reaction. The three hypervariable regions determine the specificity of the antibody, also known as complementary determining regions (CDRs). Each light-chain variable region (LCVR) and each heavy-chain variable region (HCVR) are composed of three CDRs and four FRs, with a sequential rank order from the amino terminal to the carboxyl terminal being: FR1, CDR1, FR2, CDR2, FR3, CDR3, and FR4. The three CDRs of the light chain, namely the light-chain hypervariable regions (LCDRs), refer to LCDR1, LCDR2 and LCDR3; and the three CDRs of the heavy chain, namely the heavy-chain highly variable regions (HCDRs), refer to HCDR1, HCDR2 and HCDR3. The numbers and locations of CDR amino acid residues in the LCVRs and HCVRs of the antibody or antigen-binding fragment of the present invention correspond with known Kabat numbering scheme (LCDR1-3, HCDE2-3), or correspond with kabat and chothia numbering schemes (HCDR1). The four FRs in the natural heavy-chain and light-chain variable regions are roughly in a β-folded configuration linked by three CDRs that form a linking ring, and in some cases, the four FRs may form a partially β-folded structure. The CDRs in each chain are closely brought together by the FRs, and form, together with the CDRs from another chain, an antigen-binding site of the antibody. It is possible to determine which amino acids make up the FRs or CDRs by comparing the amino acid sequences of antibodies of the same type. The constant regions are not directly involved in the binding between an antibody and an antigen, but they exhibit different effector functions, for example, participating in the antibody-dependent cytotoxicity of the antibody.

As used herein, the term "antigen-binding fragment" refers to a Fab fragment, a Fab' fragment, a F(ab')2 fragment, or a single Fv fragment, having an antigen-binding activity. The Fv antibody contains a heavy chain variable region and a light chain variable region, but does not have a constant region, and has a minimum antibody fragment with all antigen binding sites. Generally, the Fv antibody further contains a polypeptide linker between the VH and VL domains, and can form a structure required for antigen binding.

As used herein, the term "antigenic determinant" refers to three-dimensional spatial sites distributed discontinuously on an antigen and identified by the antibody or antigen-binding fragment of the present application.

The present application includes not only intact antibodies, but also fragments of immunocompetent antibodies or fusion proteins formed by antibodies with other sequences. Therefore, the present application further comprises fragments, derivatives, and analogues of the antibody.

In the present application, the antibody comprises murine, chimeric, humanized or fully human antibodies prepared with techniques familiar to those skilled in the art. Recombinant antibodies, for example, chimeric and humanized monoclonal antibodies (including human and non-human moieties), can be prepared using the recombinant DNA techniques well known in the art.

As used herein, the term "monoclonal antibody" refers to an antibody secreted by a clone derived from a single cell source. The monoclonal antibody is highly specific for a single epitope. The cells may be eukaryotic, prokaryotic, or phage clonal cell lines.

As used herein, the term "chimeric antibody" is an antibody molecule expressed by splicing the V region gene of a murine antibody and the C region gene of a human antibody into a chimeric gene, and then inserting the chimeric gene into a vector to transfect a host cell. It not only retains the high specificity and affinity of parental mouse antibody, but also enables its human-derived Fc segment to effectively mediate the biological effector functions.

As used herein, the term "humanized antibody" is a variable region modification form of the murine antibody of the present application. It has CDRs derived from (or substantially derived from) a non-human antibody (for example, which may be a mouse monoclonal antibody), and FRs and constant regions substantially derived from the human antibody sequence. That is, the CDR sequences of the murine antibody are grafted onto different types of human germline antibody framework sequences. Since the CDR sequences are responsible for most of the antibody-antigen interactions, recombinant antibodies that mimic the properties of specific naturally-occurring antibodies can be expressed by constructing expression vectors.

In the present application, the antibody may be monospecific, bispecific, trispecific, or more polyspecific.

In the present application, the antibody of the present application further comprises conserved variants thereof, which refer to polypeptides formed by substituting up to 10, for example possibly up to 8, for example possibly up to 5, and for example possibly up to 3 amino acids with amino acids having similar or resembling properties, as compared with the amino acid sequence of the antibody of the present application. These conserved variant peptides are best produced by amino acid substitution according to Table A.

**Table A**

| Initial residue | Representative substituent | Preferred substituent |
|---|---|---|
| Ala (A) | Val; Leu; Ile | Val |
| Arg (R) | Lys; Gln; Asn | Lys |
| Asn (N) | Gln: His: Lys: Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gin; Lys; Arg | Arg |
| Ile (I) | Elu; Val; Met; Ala; Phe | Leu |
| Leu (L) | Ile, Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-IL-36R antibody

As used herein, the term "IL-36R" generally refers to natural or recombinant human IL-36R, as well as non-human homologues of human IL-36R. Unless otherwise indicated, the molar concentration of IL-36R is calculated using the molecular weight of the homodimer of IL-36R.

The present application provides a high-specificity and high-affinity antibody targeting IL-36R. The antibody comprises a heavy chain and a light chain; the heavy chain comprises a heavy-chain variable region (VH) amino acid sequence, and the light chain comprising a light-chain variable region (VL) amino acid sequence.

For example, the CDR of the heavy-chain variable region (VH) is selected from the group consisting of:
CDR1 as set forth in SEQ ID NO: 3,
CDR2 as set forth in SEQ ID NO: 4, and
CDR3 as set forth in SEQ ID NO: 5; and/or
the CDR of the light-chain variable region (VL) is selected from the group consisting of:
   CDR1' as set forth in SEQ ID NO: 6,
   CDR2' as set forth in SEQ ID NO: 7, and
   CDR3' as set forth in SEQ ID NO: 8.

Here, any of the above amino acid sequences further comprises a derivative sequence which has been added, deleted, modified and/or substituted with at least one (such as 1-5, 1-3, for example possibly 1-2, for example possibly 1) amino acid and has IL-36R binding affinity.

In some embodiments, a sequence formed by the addition, deletion, modification, and/or substitution of at least one amino acid sequence may have at least 80%, for example at least 85%, for example at least 90%, or for example at least 95% sequence homology with the amino acid sequence.

The antibody of the present application may be a double-chain or single-chain antibody, and may be selected from animal-derived antibodies, chimeric antibodies, and humanized antibodies, for example, which may be a humanized antibody or a human-animal chimeric antibody, for example, a fully humanized antibody.

The antibody derivatives described in the present application may include single-chain antibodies, and/or antibody fragments (such as: Fab, Fab', (Fab')2 or other antibody derivatives known in the art), and IgA, IgD, IgE, IgG and IgM antibodies or any one or more of other subtypes of antibodies.

Here, the animal may be for example a mammal, such as a mouse.

The antibody of the present application may be a murine antibody, chimeric antibody, humanized antibody, or CDR-grafted and/or -modified antibody targeting human IL-36R.

In an exemplary embodiment of the present application, any one or more sequences of the above SEQ ID NO: 3, 4 and 5, or their sequences that have at least one amino acid added, deleted, modified and/or substituted and have IL-36R binding affinity, are located in the CDR of the heavy-chain variable region (VH).

In an exemplary embodiment of the present application, any one or more sequences of the above SEQ ID NO: 6, 7 and 8, or their sequences that have at least one amino acid added, deleted, modified and/or substituted and have IL-36R binding affinity, are located in the CDR of the light-chain variable region (VH).

In an exemplary embodiment of the present application, VH CDR1, CDR2, and CDR3 are each independently selected from any one or more sequences of SEQ ID NO: 3, 4, and 5, or their sequences that have at least one amino acid added, deleted, modified and/or substituted and have IL-36R binding affinity; and VL CDR1, CDR2, and CDR3 are each independently selected from any one or more sequences of SEQ ID NO: 6, 7, and 8, or their sequences that have at least one amino acid added, deleted, modified and/or substituted and have IL-36R binding affinity.

In the above description of the present application, the number of the amino acids added, deleted, modified and/or substituted, for example, may not exceed 40%, for example may not exceed 35%, for example may be 1-33%, for example may be 5-30%, for example may be 10-25%, or for example may be 15-20%, of the total number of amino acids in an initial amino acid sequence.

In the present application, the number of amino acids added, deleted, modified and/or substituted is usually 1, 2, 3, 4 or 5, for example, which may be 1-3, for example may be 1-2, and for example may be 1.

In the present application, the CDR sequences are partitioned according to specific CDR partitioning criteria (for example, IMGT method). It should be noted that, if CDRs obtained by partitioning an amino acid sequence of a PD-L1 antigen-binding protein according to the specific CDR partitioning criteria (for example, IMGT method) is the same as the CDR sequences involved in the present application, the amino acid sequence of the PD-L1 antigen-binding protein also falls within the scope of protection of the present application. Depending on CDR partitioning criteria, the precise identification of the CDR position may be slightly different, and therefore the present application includes not only the CDRs shown in the sequence listing, but also the CDRs included in the VH and VL domains when other numbering systems are used. In addition, these VH and VL sequences can be used in an scFv or Fab form.

In the present application, a moiety of each heavy-chain or light-chain amino acid sequence of the antigen-binding protein is homologous to the corresponding amino acid sequence in an antibody from specific species, or belong to a specific class. For example, both the heavy-chain and light-chain variable and constant moieties are derived from the variable and constant regions of an antibody of one animal species (such as human). In the present application, the homologue may be a protein or polypeptide that has at least about 85% (for example, at least about 85%, about 90%, about 91%, about 92%, about 93%, about 94%, about 95%, about 96%, about 97%, about 98%, about 99% or higher) sequence homology with the amino acid sequence of the defined protein and/or polypeptide (for example, the antibody specifically binding to the I-36R protein, or a fragment thereof).

In the present application, the homology generally refers to the similarity, resemblance or association between two or more sequences. The alignment for determining the sequence homology percentage may be implemented in a variety of ways known in the art, for example, by using publicly available computer software such as BLAST, BLAST-2, ALIGN or Megalign (DNASTAR) software. Those skilled in the art may determine appropriate parameters for sequence alignment, including any algorithm required to implement the maximum alignment undergoing comparison, within a full-length sequence range or within a target sequence region. The homology may also be determined by the following methods: FASTA and BLAST. The description of the FASTA algorithm can be found in W.R. Pearson and D. J. Lipman "Improved Tools for Biological Sequence Comparison", Proc. Natl. Acad. Sci., 85: 2444-2448, 1988; and D.J.Lipman and W.R. Pearson "Rapid and Sensitive Protein Similarity Searches", Science, 227: 1435-1441, 1989. The description of the BLAST algorithm can be found in S. Altschul, W. Gish, w. Miller, E. W. Myers and D. Lipman, "Basic Local Alignment Search Tool", J. Mol. Biol., 215: 403-410, 1990.

### Polyspecific Antibody

The polyspecific antibody, comprising a first targeting moiety capable of specifically binding to an IL-36R protein, wherein the first targeting moiety comprises an isolated antigen-binding protein comprising a heavy-chain variable region (VH), which comprises the following three complementarity determining regions (CDRs):
CDR1 as set forth in SEQ ID NO: 3,
CDR2 as set forth in SEQ ID NO: 4, and
CDR3 as set forth in SEQ ID NO: 5.

In some embodiments, any of the above amino acid sequences further comprises a derivative sequence, which has at least one (such as 1 to 3, for example, possibly 1 to 2, for example, possibly 1) amino acid optionally added, deleted, modified and/or substituted and can retain the IL-36R binding affinity.

In some embodiments, the heavy-chain variable region further comprises a human-derived FR or a murine FR.

In some embodiments, the heavy-chain variable region has an amino acid sequence as set forth in SEQ ID NO: 1.

In some embodiments, the heavy-chain variable region has an amino acid sequence as set forth in SEQ ID NO: 9.

In another aspect of the present application, the polyspecific antibody comprises a light-chain variable region (VL), which comprises
the following three complementary determining regions (CDRs):
CDR1' as set forth in SEQ ID NO: 6,
CDR2' as set forth in SEQ ID NO: 7, and
CDR3' as set forth in SEQ ID NO: 8.

In some embodiments, any of the above amino acid sequences further comprises a derivative sequence, which has at least one (such as 1 to 3, for example, possibly 1 to 2, for example, possibly 1) amino acid optionally added, deleted, modified and/or substituted and can retain the IL-36R binding affinity.

In some embodiments, the light-chain variable region further comprises a human-derived FR or a murine FR.

In some embodiments, the light-chain variable region has an amino acid sequence as set forth in SEQ ID NO: 2.

In some embodiments, the light-chain variable region has an amino acid sequence as set forth in SEQ ID NO: 10, 11, or 12.

In some embodiments, the polyspecific antibody further comprises a second targeting moiety, for example, which may specifically bind to IL-17A.

### Bispecific Antibody

As used herein, the terms "bispecific antibody", "bifunctional antibody", "bi-antibody of the present application", "bi-antibody" and "bifunctional fusion antibody" can be used interchangeably, and refer to the bispecific antibody capable of simultaneously targeting IL-17A and IL-36R according to the sixth aspect of the present application.

In an exemplary embodiment, the bispecific antibody is fused from the anti-IL-17A antibody and the ScFv of the anti-IL-36R antibody, and has two pairs of peptide chains symmetrical to each other, each pair of the peptide chains comprises a light chain L-chain and a heavy chain H-chain, and all the peptide chains are linked by the disulfide bonds, wherein any pair of the peptide chains have the structures of the H-chain and the L-chain from the N-terminus to the C-terminus as set forth in Formula I. 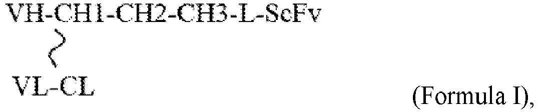 wherein,
VH represents the heavy-chain variable region of the anti-IL-17A antibody;
VL represents the light-chain variable region of the anti-IL-17A antibody;
CHI, CH2 and CH3 represent the heavy-chain constant regions CH1, CH2 and CH3 of the IL-17A antibody, respectively;
CL represents the light-chain constant region of the anti-IL-17A antibody;
ScFv represents the ScFv of the anti-IL-36R antibody;
L indicates a linker element;
"~" represents a disulfide bond;
"-" represents a peptide bond; and
wherein the bispecific antibody has an activity of simultaneously binding to the IL-17A and the IL-36R.

For example, the H-chain of the bispecific antibody may have an amino acid sequence as set forth in anyone of SEQ ID NO: 13 to 18; and the L-chain of the bispecific antibody has an amino acid sequence as set forth in SEQ ID NO: 19.

The bi-antibody of the present application includes not only intact antibodies, but also fragments of immunocompetent antibodies or fusion proteins formed by antibodies with other sequences. Therefore, the present application further comprises fragments, derivatives, and analogues of the antibody.

The bi-antibody of the present application refers to an antibody having anti-IL-17A and anti-IL-36R activities and including two structures of the above Formula I. The term also includes variants of the antibody having the same function as the bi-antibody of the present application and including two structures of the above Formula I. These variants include (but are not limited to): deletion, insertion and/or substitution of one or more (typically 1-50, for example possibly 1-30, for example possibly 1-20, for example possibly 1-10) amino acids, and addition of one or more (typically within 20, for example possibly within 10, for example possibly within 5) amino acids at the C-terminus and/or N-terminus. For example, in the art, the function of a protein is typically not changed when amino acids with similar or similar properties are used for substitution. For another example, adding one or more amino acids to the C-terminus and/or N-terminus typically does not change the function of the protein. The term also includes active fragments and active derivatives of the bi-antibody of the present application.

### Preparation of Antibodies

Any method suitable for producing a monoclonal antibody can be used to produce the IL-36R antibody of the present application. For example, animals can be immunized with linked or naturally occurring IL-36R homodimers or fragments thereof. Suitable immunization methods may be used, including adjuvants, immunostimulants, and repeated booster immunizations, and one or more routes may be used.

Any suitable form of IL-36R may be used as an immunogen (antigen) to produce a non-human antibody specific to IL-36R and to screen the bioactivities of the antibody. An eliciting immunogen may be a full-length mature human IL-36R, comprising natural homodimers, or peptides containing single/multiple epitopes. The immunogen may be used alone, or in combination with one or more immunogenicity enhancers known in the art. The immunogen may be purified from a natural source, or produced in a genetically modified cell. An DNA encoding the immunogen may be genomic or non-genomic (for example, cDNA) in origin. A suitable genetic vector may be used to express the DNA encoding the immunogen, and the vector comprises, but is not limited to, an adenovirus vector, an adeno-associated virus vector, a baculovirus vector, a material, and a non-viral vector.

An exemplary method for producing the anti-human IL-36R antibody of the present application is described in Example 1.

A humanized antibody may be selected from any class of immunoglobulin, including IgM, IgD, IgG, IgA, and IgE. In the present application, the antibody is an IgG antibody, and an IgG1 subtype is used. With the biological assays described in the examples below, it is easy to optimize an essential constant domain sequence by screening antibodies, so as to produce the desired bioactivity.

Similarly, any type of light chain can be used in the compounds and methods herein. Specifically, κ and λ chains or their variants can be used in the compounds and methods of the present application.

An exemplary method for humanizing the anti-human IL-36R antibody of the present application is described in Example 2.

The sequence of the DNA molecule of the antibody or the fragment thereof in the present application can be obtained by conventional techniques, such as methods using PCR amplification or genomic library screening and the like. In addition, the coding sequences of the light and heavy chains may also be fused together to form a single-chain antibody.

Once relevant sequences are obtained, they may be obtained on a large scale by recombination. This is generally done by cloning them into vectors, then transferring then into cells, and then isolating the relevant sequences from the proliferated host cell by means of a conventional method.

In addition, the relevant sequences may also be synthesized by using an artificial synthesis method, in particular when a fragment is short. Generally, a fragment with a very long sequence may be obtained by first synthesizing multiple small fragments, and then linking these small fragments. Then, this DNA sequence may be introduced into various existing DNA molecules (or such as vectors) and cells known in the art.

The present application further relates to vectors comprising the aforementioned appropriate DNA sequences and appropriate promoters or control sequences. These vectors may be used to transform appropriate host cells to enable them to express proteins.

The host cells may be prokaryotic cells, such as bacterial cells; or lower eukaryotic cells, such as yeast cells; or higher eukaryotic cells, such as mammalian cells. For example, the animal cells may comprise (but are not limited to): CHO-S, CHO-K1, and HEK-293 cells.

The step of transforming the host cells with recombinant DNAs in the present application may be performed using techniques well known in the art. A resulting transformant may be cultured by a conventional method, and it expresses the polypeptide encoded by the gene of the present application. According to the host cells used, they are cultured in a conventional medium under suitable conditions.

Usually, the host cells are cultured and transformed under conditions suitable for the expression of the antibody of the present application. Then, the antibody of the present application is purified and obtained using conventional immunoglobulin purification steps, such as protein A-Sepharose, hydroxyapatite chromatography, gel electrophoresis, dialysis, ion exchange chromatography, hydrophobic chromatography, molecular sieve chromatography, or affinity chromatography, and other conventional separation and purification means well known to those skilled in the art.

The resulting monoclonal antibody may be identified by a conventional means. For instance, the binding specificity of the monoclonal antibody may be determined by immunoprecipitation or *in vitro* binding assays (such as radioimmunoassay (RIA) or enzyme-linked immunosorbent assay (ELISA)).

### Antibody-Drug Conjugate (ADC)

The present application further provides an antibody-drug conjugate (ADC) based on the antibody of the present application.

Typically, the antibody-drug conjugate comprises the antibody, and an effector molecule. The antibody is conjugated (for example, chemically conjugated) to the effector molecule. For example, the effector molecule may be a drug having a therapeutic activity. Furthermore, the effector molecule may be one or more of a toxic protein, a chemotherapy drug, a small molecule drug, or a radionuclide.

The antibody of the present application and the effector molecule may be conjugated with each other by a conjugating agent. Examples of the conjugating agent may be any one or more of a non-selective conjugating agent, a conjugating agent using a carboxyl group, a peptide chain, a conjugating agent using a disulfide bond. The non-selective conjugating agent refers to a compound such as glutaraldehyde, which allows the effector molecule and the antibody to be linked via a covalent bond. The conjugating agent using the carboxyl group may be any or more of a cis- aconitic anhydride-based conjugating agent (for example, cis-aconitic anhydride), an acylhydrazone-based conjugating agent (with acylhydrazone as a conjugating site).

Some residues (such as Cys or Lys) on the antibody are used to link a variety of functional groups, involving an imaging reagent (for example, a chromophore and a fluorophore), a diagnostic reagent (for example, an MRI contrast agent and a radioisotope), a stabilizer (for example, a glycol polymer), and a therapeutic agent. The antibody may be conjugated to a functional agent to form an antibody-functional agent conjugate. The functional agent (for example, a drug, a detection reagent, or a stabilizer) is conjugated (covalently linked) to the antibody. The functional agent may be attached to the antibody directly or indirectly via a linker.

The antibody may conjugate a drug to form an antibody-drug conjugate (ADC). Typically, the ADC comprises a linker between the drug and the antibody. The linker may be degradable or nondegradable. The degradable linker typically degrades easily in an intracellular environment, for example, at a site of interest, to release the drug from the antibody. Suitable degradable linkers include, for example, enzymatically degradable linkers, including peptidyl-containing linkers degradable by intracellular proteases (for example, lysosomal proteases or endosomal proteases); or saccharide linkers, for example, glucuronide-containing linkers degradable by glucuronidases. The peptidyl-containing linkers may include, for example, dipeptides, such as valine-citrulline, phenylalanine-lysine, or valine-alanine. Other suitable degradable linkers include, for example, pH-sensitive linkers (for example, linkers hydrolyzed at pH less than 5.5, for example, hydrazone linkers) and linkers (for example, disulfide bond linkers) degrading under reducing conditions. Nondegradable linkers typically release drugs under conditions where antibodies are hydrolyzed by proteases.

Before linking the antibody, the linker has an active reactive group capable of reacting with some amino acid residues, and the linkage is achieved by the active reactive group. For example, sulfhydryl-specific active reactive groups may be and include: for example, maleimide-based compounds, halogenated amides (for example, iodo-, bromo- or chlorinated amides); halogenated esters (for example iodo-, bromo- or chlorinated esters); halogenated methylketones (for example iodo-, bromo- or chlorinated methylketones), benzyl halides (for example iodides, bromides or chlorides); vinyl sulfone, pyridyl disulfide; mercury derivatives, for example, 3,6-bis-(mercury methyl)dioxane, with acetates, chloride ions or nitrates as p-ions; and polymethylene dimethyl sulfide thiosulfonates. The linkers may include, for example, maleimide attached to the antibody by thiosuccinimide.

The drug may be any cytotoxic, cell-growth inhibitory, or immunosuppressive drug. In an embodiment, the linker links the antibody and the drug, and the drug has a functional group that can be bonded with the linker. For example, the drug may have an amino, carboxyl, sulfhydryl, hydroxyl, or ketone group that can be bonded to the linker. In the case where the drug is directly linked to the linker, the drug has a reactive active group before linking the antibody.

Useful drug categories include, for example, antitubulin drugs, DNA minor groove binding reagents, DNA replication inhibitors, alkylation reagents, antibiotics, folate antagonists, antimetabolites, chemosensitizers, topoisomerase inhibitors, vinca alkaloids, etc. In the present application, a drug-linker may be used to form an ADC in a simple step. In other embodiments, a bifunctional linker compound may be used to form an ADC in a two-step or multi-step method. For example, a cysteine residue reacts with the reactive portion of the linker in a first step, and in subsequent steps, the functional group on the linker reacts with the drug, thereby forming an ADC.

Usually, the functional group on the linker is selected to facilitate specific reaction with an appropriate reactive group on the drug moiety. As a non-limiting example, an azide-based moiety may be used to react specifically with a reactive alkyne group on the drug moiety. The drug is covalently bonded to the linker by addition of a 1,3-dipole ring between azide and alkyne. Other useful functional groups include, for example, ketones and aldehydes (suitable for reacting with hydrazides and alkoxyamines), and phosphines (suitable for reacting with azides); isocyanates and isothiocyanates (suitable for reacting with amines and alcohols); and activated esters, for example N-hydroxysuccinimidyl esters (suitable for reacting with amines and alcohols). These and other linkage strategies (for example, those described in Bioconjugation Techniques, Second Edition (Elsevier)) are well known to those skilled in the art. Those skilled in the art can understand that, for the selective reaction between the drug moiety and the linker, when a complementary pair of reactive functional groups is selected, each member in the complementary pair may be used both for the linker and for the drug.

The present application further provides a preparation method for an ADC, further comprising: binding an antibody with a drug-linker compound under conditions sufficient to form an antibody conjugate (ADC).

In some embodiments, the method of the present application may comprise: binding the antibody to a bifunctional linker compound under conditions sufficient to form an antibody-linker conjugate. In these embodiments, the method of the present application may further comprise: binding the antibody-linker conjugate to the drug moiety under conditions sufficient to covalently link the drug moiety to the antibody via the linker.

In some embodiments, the antibody-drug conjugate (ADC) is as shown in the following molecular formula:

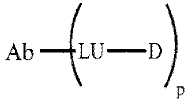

Here,
Ab indicates an antibody,
LU indicates a linker;
D indicates a drug;
and the subscript p is a value selected from 1 to 8.

### Application

The present application further provides a use of the antibody of the present application, for example, for the preparation of diagnostic preparations, or the preparation of drugs for the prevention and/or treatment of IL-36-related diseases. The IL-36 related diseases comprise inflammatory diseases, autoimmune diseases, etc., including but not limited to psoriasis, psoriatic arthritis, ankylosing spondylitis, multiple sclerosis, inflammatory bowel disease (such as Crohn's disease and ulcerative colitis), osteoarthritis, rheumatoid arthritis (RA), rheumatic arthritis or osteoporosis, inflammatory fibrosis (for example, scleroderma, and pulmonary fibrosis and sclerosis), asthma (including allergic asthma), allergic reactions, and cancers.

### Pharmaceutical composition

The present application further provides a composition. For example, the composition is a pharmaceutical composition, which comprises the antigen-binding protein described above, or an active fragment thereof, or a fusion protein thereof, or an ADC thereof, or corresponding CAR-T cells, and a pharmaceutically acceptable carrier. Usually, these substances may be formulated in a non-toxic, inert, and pharmaceutically acceptable aqueous carrier medium, of which the pH is usually about 5-8, for example about 6-8., although the pH value may vary with the properties of the formulated substances and the disorders to be treated. The formulated pharmaceutical composition may be administered by conventional routes, comprising (but not limited to): intratumoral, intraperitoneal, intravenous, or topical administration.

The antibody of the present application may also be expressed in a cell by means of a nucleotide sequence for cell therapy. For example, the antibody is used for the chimeric antigen receptor T cell immunotherapy (CAR-T), etc.

The pharmaceutical composition of the present application may be directly used to bind an IL-36R protein molecule, and thus may be used to prevent and treat IL-36R-related diseases. In addition, other therapeutic agents may also be used at the same time.

The pharmaceutical composition of the present application comprises the aforementioned monoclonal antibody (or a conjugate thereof) of the present application at a safe and effective amount (such as 0.001-99 wt%, for example possibly 0.01-90 wt%, for example possibly 0.1-80 wt%) and pharmaceutically acceptable carriers or excipients. Such carriers comprise (but are not limited to): saline water, buffer, glucose, water, glycerol, ethanol, and combinations thereof. A pharmaceutical preparation should match the mode of administration. The pharmaceutical composition of the present application may be prepared into an injection form, for example, by means of a conventional method using normal saline or an aqueous solution containing glucose and other adjuvants. The pharmaceutical composition such as an injection and a solution should be manufactured under an aseptic condition. The dosage of an active ingredient is a therapeutically effective amount, for example, about 1 µg/kg body weight to about 5 mg/kg body weight per day. Furthermore, the polypeptide of the present application may also be used together with other therapeutic agents.

When in use, the pharmaceutical composition is administered to mammals at a safe and effective amount, which is usually at least about 10 µg/kg body weight and in most cases, does not exceed about 50 mg/kg body weight. For example, its dosage may be about 10 µg/kg body weight to about 20 mg/kg body weight. Without doubt, the specific dosage should also consider factors such as the route of administration and the health condition of a patient, which are within the scope of skills of a proficient physician.

### Detection uses and kit

The antibody of the present application can be used in detection applications (for example, for sample detection), so as to provide diagnostic information.

In the present application, the samples (specimens) used comprise cells, tissue samples, and biopsy samples. The term "biopsy" used in the present application shall comprise all types of biopsy known to those skilled in the art. Therefore, the biopsy used in the present application may comprise, for example, tissue samples prepared by an endoscopic method or by organ puncture or needle biopsy.

The samples used in the present application comprise fixed or preserved cell or tissue samples.

The present application further provides a kit comprising the antibody (or a fragment thereof) of the present application. In an exemplary embodiment of the present application, the kit further comprise a container, an instruction manual, a buffer, etc. In an exemplary embodiment, the antibody of the present application may be fixed on a detection plate.

The main advantage of the present application includes:
(a) the antibody of the present application has excellent bioactivity and specificity.
(b) Compared with a murine antibody and a chimeric antibody, the humanized antibody of the present application has lower immunogenicity while retaining a comparable affinity with IL-3 6R.
(c) The blocking of IL-36R by the antibody of the present application can significantly inhibit the inflammatory factor expression pathway in a patient.
(d) The antibody of the present application has a comparable affinity with some non-human mammals (such as monkeys) IL-36R as with human IL-36R, which facilitates testing and quality control detection in animal models.
(e) The antibody of the present application binds to IL-36R to block the activation signaling pathway for binding IL-36α, IL-36β, or IL-36γ to IL-36R, thereby reducing inflammatory cytokines (such as IL-6, IL-8, or GM-CSF).

The present application is further set forth below in conjunction with specific examples. It should be understood that these embodiments are intended only to illustrating the present application, instead of limiting the scope of the present application. Experimental methods without specific conditions indicated in the following examples usually follow conventional conditions, for example the conditions described in Sambrook et al., Molecular Cloning: A Laboratory Manual (New York: Cold Spring Harbor Laboratory Press, 1989), or according to conditions recommended by the manufacturers. Unless otherwise specified, percentages and parts are weight percentages and parts by weight.

### Examples

### Example 1 Preparation of Hybridoma Cells for Production of Murine Monoclonal Antibodies

The method for preparing murine monoclonal antibodies was based on the hybridoma preparation technology invented by Kohler and Milstein in 1975 (Nature, 1975, 256: 495-497). First, Fc-tagged proteins (ACRO, #IL2-H5254) of human IL-36R were emulsified with the Freund's adjuvant, and then used to immunize multiple strains of mice. After four rounds of immunizations, the serum was collected to detect the titer by ELISA, and spleen cells were harvested from the selected optimal mice for fusion with SP2/0 myeloma cells. After HAT screening of hybridoma polyclonal cells, a polyclonal culture supernatant was tested in the binding activities of human IL-36R, monkey IL-36R and human IL1R1, and the functional inhibition effects of polyclonal antibodies against IL-36 stimulating factors were tested. The best polyclones were picked for monoclonalization, the resulting monoclones were also screened in binding activity and functional activity, and the affinity was detected by the Biacore method to finally select the best hybridoma monoclonal cell strains for sequence analysis.

**Table 1. Hybridoma screening data in part**

| Clone name | Binding activity OD450 value | MFI ratio for binding activity to human IL-36R (hybridoma supernatant/ positive control) | MFI ratio for binding activity to monkey IL-36R (hybridoma supernatant/ positive control) | MFI ratio for binding activity to human IL1R1 (hybridoma supernatant/ negative control) | IL-6 ratio in blockage of human IL36β functional activity (hybridoma supernatant/ positive control) | IL-8 ratio in blockage of human IL36α functional activity (hybridoma supernatant/ positive control) | IL-8 ratio in blockage of human IL36β functional activity (hybridoma supernatant/ positive control) | IL-8 ratio in blockage of human IL36γ functional activity (hybridoma supernatant/ positive control) |
|---|---|---|---|---|---|---|---|---|
| 6(1) -B8 | 1.293 | 75.66% | 94.41% | 120.18% | 10.50% | 4.78% | 5.08% | 5.77% |
| 6 (2) -G8 | 1.74 | 94.54% | 81.50% | 114.91% | 13.18% | 5.61% | 6.45% | 5.58% |
| 21 (1) -E4 | 0.943 | 67.89% | 90.03% | 122.81% | 5.70% | -3.19% | -1.97% | -2.50% |
| 21 (2)-F3 | 1.681 | 80.91% | 91.36% | 114.91% | 1.18% | -2.61% | -1.88% | -2.31% |
| 65 (1)-B5 | 1.355 | 56.84% | 85.44% | 111.40% | 23.05% | 15.66% | 18.02% | 12.87% |
| 65 (2) -F11 | 1.319 | 71.60% | 88.52% | 116.67% | 21.66% | 15.90% | 17.72% | 13.17% |
| 74 (1) -E2 | 1.917 | 109.76% | 85.93% | 114.04% | 1.00% | -3.11% | -3.15% | -2.89% |
| 74 (2)-G5 | 1.827 | 103.89% | 97.44% | 114.91% | 2.84% | -2.86% | -2.66% | -2.60% |
| 79 (1) -C7 | 2.133 | 112.13% | 100.76% | 110.53% | 13.08% | 3.12% | 3.51% | 3.34% |
| 79(2)-G7 | 1.085 | 75.48% | 102.59% | 121.93% | 8.75% | 4.70% | 1.46% | 3.24% |
| 84 (1)-C11 | 2.277 | 84.52% | 86.85% | 115.79% | 0.90% | -3.27% | -3.35% | -2.99% |
| 84 (2)-C3 | 2.324 | 64.61% | 104.35% | 114.91% | 0.81% | -4.02% | -3.05% | -3.47% |
| 111 (1)-E5 | 2.161 | 81.64% | 105.10% | 118.42% | 0.07% | -4.02% | -4.62% | -3.86% |
| 111 (2)-B3 | 2.36 | 102.30% | 103.62% | 116.67% | 0.07% | -4.68% | -4.62% | -4.06% |
| 130(1) -H7 | 1.316 | 113.29% | 110.61% | 119.30% | 30.89% | 44.55% | 26.64% | 25.52% |
| 130 (2) -A2 | 1.507 | 83.19% | 130.12% | 123.68% | 31.35% | 43.22% | 27.13% | 22.31% |
| 140 (1) -C1 | 1.768 | 84.57% | 102.87% | 117.54% | 1.92% | -4.35% | -3.74% | -3.09% |
| 172 (1) -H4 | 1.442 | 95.40% | 96.88% | 114.91% | 44.92% | 58.91% | 46.82% | 68.93% |
| 172 (2)-C4 | 1.814 | 82.50% | 89.66% | 116.67% | 45.10% | 55.75% | 46.73% | 67.56% |
| 179 (1)-G7 | 1.667 | 74.17% | 113.09% | 114.04% | 30.06% | 27.19% | 24.48% | 18.32% |
| 180 (1) -H5 | 2.082 | 81.42% | 79.75% | 116.67% | 9.48% | 0.21% | 1.46% | -1.63% |
| 180 (2) -D3 | 2.477 | 65.03% | 86.87% | 114.91% | 13.54% | 3.54% | 4.89% | 1.78% |
| 181 (2) -F10 | 2.959 | 49.74% | 109.08% | 126.32% | 20.65% | 15.07% | 12.53% | 10.15% |
| 182 (1) -C8 | 1.843 | 93.33% | 86.26% | 120.18% | 50.27% | 74.76% | 57.11% | 55.89% |
| 183 (1) -H7 | 2.016 | 84.65% | 84.83% | 115.79% | 30.89% | 26.86% | 23.99% | 22.31% |
| 183 (2)-F12 | 1.857 | 59.34% | 84.47% | 127.19% | 24.71% | 18.23% | 18.90% | 14.53% |
| 184 (1) -F8 | 2.27 | 96.60% | 86.57% | 118.42% | 0.26% | -3.60% | -5.11% | -4.06% |
| 184 (2) -B9 | 2.408 | 98.36% | 89.91% | 120.18% | -1.80% | -4.45% | -7.19% | -3.44% |

As shown in Table 1, after extensive hybridoma screening, the antibody expression supernatants of hybridomas 6(1)-B8 and 111(1)-E5 showed very high binding activity to human IL-36R and monkey IL-36R proteins at a cell binding level, but no specific binding to human II,1R1 proteins, and showed an obvious functional blocking effect on cytokines produced by the stimulation of IL-36 stimulating factors (IL-36α, IL-36β, and IL-36γ).

### Example 2 Cloning and Humanization of Variable Region Gene Sequences of Anti-IL-36R Antibodies

### 2.1 Cloning of antibody variable region genes in hybridoma cells

Based on the principle of TAKARA's 5' RACE technology, the cDNA sequences of the variable regions of the mouse antibody expressed by the hybridoma cell strains were cloned. Briefly, the variable region gene-specific cDNAs of the heavy and light chains were synthesized by using the SMARTer 5'RACE synthesis kit (TAKARA, Cat. #:634859) following the instructions. The 5'- and 3'-termini of the cDNA sequences were modified with PCR primers, which were designed to add appropriate leader sequences to the variable region cDNAs of the heavy and light chains, respectively, such that the resulting PCR products could be cloned, by a seamless cloning method, onto a heavy-chain vector pHB-Fc and a light-chain vector pHB-Cκ which were expressed in the existing recombinant antibody. The expression vector pHB-Fc contained the human IgG1 heavy-chain constant region gene sequence, with L234A and L235A (Eu numbering) mutations having weakened antibody ADCC effects on CH2; and the vector pHB-Cκ contained the human κ light-chain constant region gene sequence. The PCR amplification products of the heavy- and light-chain variable regions were cloned into the expression vectors by means of an In-fusion cloning reagent (TAKARA, Cat. #: 639650) to obtain human-mouse chimeric antibody expression vectors, which were then transformed into *E. coli* DH5α competent cells (Yeastern Biotech, Cat. #: FYE607-80VL). Monoclonal colonies were picked for Sanger sequencing, and antibody variable region sequences were obtained by analysis.

As a result, anti-IL-36R chimeric antibodies (HUABO No.: 900497) was obtained, with the light chain derived from the hybridoma 6(1)-B8 in Example 1, and the heavy chain derived from the hybridoma 111(1)-E5 in Example 1. The sequences of its variable regions were as follows:
900497 HCVR SEQ ID NO: 1 
QVQLQQTGSVLVRPGTSVKLSCKASGYTFTSSWMHWAKQRPGQGLEWIGEIHPNSAKT
NYNEKFKGKATLTVDTFSSTAYVDLSSLASEDSAVYYCARVDYGKPWFAYWGQGTLVT
VSA
900497 LCVR SEQ ID NO: 2

QIVLTQSPTIMSASPGERVTMTCSASSSVSSSYLHWYQQKPGSSPKLWIYSTSNLASGVPA
RFSGSGSGTSYSLTINSMEAEDAATYYCQQFQSSPLTFGAGTKLGLK

Here, the underlined regions were CDRs (as defined by IMGT, the single sequences were as follows):

**Table 2. CDR sequences of murine anti-IL-36R antibodies**

| Domain | | Sequence | SEQ ID NO: |
|---|---|---|---|
| VH | CDR1 | GYTFTSSW | 3 |
| | CDR2 | IHPNSAKT | 4 |
| | CDR3 | ARVDYGKPWFAY | 5 |
| VL | CDR1 | SSVSSSY | 6 |
| | CDR2 | STS | 7 |
| | CDR3 | QQFQSSPLT | 8 |

### 2.2 Expression of chimeric antibodies

The expression vectors obtained in 2.1 were amplified by means of *Escherichia coli,* and a sufficient amount of plasmids were prepared using an endotoxin-free plasmid extraction kit (Tiangen Biotech (Beijing) Co., Ltd., Cat. #: DP117) for transient transfection to express the chimeric antibody. CHO-S cells (ThermoFisher, Cat. #: R80007) were used as host cells for expression. The two prepared heavy-chain vectors, respectively with the light-chain vectors, were mixed with polyetherimide (PEI, Polysciences, Cat. #: 24765-1) to form liposome complexes for transfecting the CHO-S cells, which were then cultured for 5-7 days in a carbon dioxide shaker. The cell culture supernatant was collected through centrifugation, and purified by a Protein A affinity chromatographic column to obtain a human-mouse chimeric antibody.

### 2.3 Humanization of murine anti-human IL-36R antibody: preparation method for humanized antibodies

The antibodies were humanized by aligning the variable region sequence of the chimeric antibody (HUABO Number: 900497) with available sequences in the NCBI IgBlast database, and carrying out identification and analysis to finally determine a human framework region (FR) suitable for constructing CDR-grafted heavy and light chains thereon.

During transformation, transformation sites were designed according to the conserved amino acid residues in the FR of the human antibody and the important amino acid residues in the FR of the antibody; the variable regions of the heavy and light chains of the chimeric antibodies were respectively designed for humanized mutations; and humanized point-mutation antibody expression plasmids were amplified and constructed using PCR. The humanized point-mutation antibody expression plasmids were expressed in the CHO-S cells and then purified respectively to obtain humanized antibody proteins. With Biacore and methods for detecting cell bioactivity and the like, the affinity of humanized antibodies, activated cytokine release and other indicators were screened to obtain three humanized anti-IL-36R antibodies with excellent performance. The resulting humanized anti-IL-36R antibodies were numbered as 900513, 900527, and 900534, and their VH and VL sequences were shown as follows:
900513, 900527 and 900534 HCVRs. SEQ ID NO: 9
900513 LCVR SEQ ID NO: 10 900527 LCVR SEQ ID NO:11 900534 LCVR SEQ ID NO:12

QIVLTQSPGTLSLSPGERATLSCRASSSVSSSYLHWYQQKPGQAPRLLIYSTSSRATGIPDR
FSGSGSGTDFTLTISRLEPEDFATYYCQQFQSSPLTFGQGTKLEIK

Here, the underlined regions were CDRs (defined according to IMGT), and 900513, 900527, and 900534 were the numbers for the three humanized antibody proteins respectively. The CDRs of humanized antibodies 900513, 900527 and 900534 were the same as those of the chimeric antibody 900497, and the FRs of humanized antibodies were mutated on the basis of chimeric 900497.

### Example 3 Bioactivity Assay of Anti-Human IL-36R Humanized Antibodies

Test materials and instruments:
His-tagged human IL-1 Rrp2/IL-1R6 proteins, ACRO Biosystems, IL2-H52H6
HBS-EP+(10X), GE, BR-1006-69
Series S Sensor Chip CM5, GE, 29-1275-56
His Capture Kit, GE, 28995056
BIACORE, GE, Biacore 8K
Glycine solution with pH 1.5, GE, BR100354

The test method was as follows.

Series S Sensor Chip CM5 was equilibrated for 20-30 min at room temperature, and then fitted in the instrument. Following the instruction manual for His Capture Kit, an anti-His antibody was immobilized onto the Series S Sensor Chip CM5. An HBS-EP+ (10X) solution was diluted 10-fold in ultrapure water as the running buffer. An antigen (His-tagged human IL-1 Rrp2/IL-IR6 protein) was diluted to 1 µg/ml and 10 µL/min using the running buffer, and loaded during 15 sec to capture around 50 RU of antigen. An antibody sample were diluted to 30 nM, 15 nM, 7.5 nM, 3.75 nM, 1.875 nM, 0.9375 nM, and 0.46875 nM using an equilibration buffer; the running buffer was taken as zero concentration; at 30 µL/min, binding and dissociation were allowed to occur for 120 s and 900 s, respectively; and the chip was regenerated with the glycine solution at pH 1.5. The sample was analyzed by a capture-based single-cycle kinetics program; the corresponding analysis program was selected to analyze the data, and it was confirmed that there was no obvious reference binding; and the Kinetics 1:1 binding model was selected for fitting analysis to acquire the kinetic parameters of the sample.

**Table 3 Affinity assay of candidate anti-human IL-36R humanized antibodies**

| Protein number | ka (1/Ms) | kd (1/s) | KD(M) |
|---|---|---|---|
| 900513 | 9.04E+05 | 1.34E-05 | 1.48E-11 |
| 900527 | 9.67E+05 | 4.37E-05 | 4.52E-11 |
| 900534 | 8.81E+05 | 5.10E-05 | 5.79E-11 |
| 900497 | 6.60E+05 | 1.68E-05 | 2.54E-11 |
| 900389 | 7.39E+05 | 1.17E-05 | 1.58E-11 |

| | | | |
|---|---|---|---|
| Note: 900389, as an anti-human IL-36R antibody developed by BI, was obtained through independent expression by HUABO who synthesized a variable region gene from sequences recorded according to the patent WO2013/074569A1. Specifically, the heavy-chain variable region of 900389 was 81B4vH33_90vH as set forth in SEQ ID NO: 89 in WO2013/074569A1, the light-chain variable region of 900389 was 81B4vK32_105vK as set forth in SEQ ID NO: 77 in WO2013/074569A1, and both 81B4vH33_90vH and 81B4vK32_105vK were obtained by carrying out humanization on the basis of a murine antibody 81B4; and the constant region of 900389 was the same as those of the candidate antibodies of the present application. | | | |

The affinity constant (KD(M)) for the binding of each humanized antibody to human IL-1 Rrp2 was shown in Table 3. The results showed that the affinity of the humanized monoclonal antibodies of the present application were close to the order of magnitude of 10-11, showing a very strong affinity.

### 3.2 Detection of functional inhibition effect of anti-human IL-36R antibody on huIL-36 stimulating factor

A human ovarian cancer cell line NCI/ADR-RES was added to a 96-well cell culture plate at 100 µL per well (4.5×10⁴ cells per well), and incubated overnight at 37°C with 5% CO₂; a series of samples to be detected was diluted and added at 50 µL/well; the plate was incubated for 15 min at 37°C with 5% CO₂; ligands huIL-36α (1 µg/mL), huIL-36β (80 µg/mL), and huIL-36γ (120 µg/mL) were added at 50 µL/well, respectively, and mixed well; and the plate was incubated for 18-24 h at 37°C with 5% CO₂. Cell culture supernatants were obtained through centrifuging and detected with the Biolegend ELISAkit as follows: huIL-6 (diluted at 1:100, Cat. #: 430503), huIL-8 (diluted at 1:5, Cat. #: 431503), and huGM-CSF (diluted at 1:2, Cat. #: 432003). The activation of NF-κB in NCI/ADR-RES cells was tested under huIL-36β stimulation as follows: the ligand huIL-36β was diluted to 240 ng/mL, added to cells pre-incubated with the antibody to be detected, and incubated for 30 min at 37°C with 5% CO₂. Supernatants were obtained through centrifugation, and after cell lysis, lysate was taken and subjected to NF-κB phosphorylation assay with a kit (Cisbio, Cat. #: 64NFBPEG).

**Table 4 Results of bioactivity assay for humanized candidate antibodies**

| Stimulating factor | Detection index /IC50 (ng/mL) | 900389 | 900513 | 900527 | 900534 | 900497 |
|---|---|---|---|---|---|---|
| IL-36α | IL-6 | 21.85 | 12.40 | 15.03 | 21.58 | 33.22 |
| | IL-8 | 57.45 | 39.78 | 31.53 | 30.39 | 43.91 |
| | GM-CSF | 33.17 | 20.65 | 22.00 | 20.17 | 32.83 |
| IL-36β | IL-6 | 24.59 | 22.34 | 13.92 | 16.80 | 20.39 |
| | IL-8 | 34.44 | 22.09 | 22.27 | 21.97 | 29.21 |
| | GM-CSF | 26.91 | 18.40 | 17.36 | 18.65 | 22.36 |
| IL-36γ | IL-6 | 26.44 | 12.05 | 16.18 | 17.61 | 19.78 |
| | IL-8 | 33.71 | 18.00 | 19.81 | 17.95 | 28.37 |
| | GM-CSF | 32.67 | 17.87 | 19.81 | 20.97 | 28.75 |
| IL-36β | pNF-κB | 179.00 | 105.30 | 71.44 | 64.92 | 115.10 |

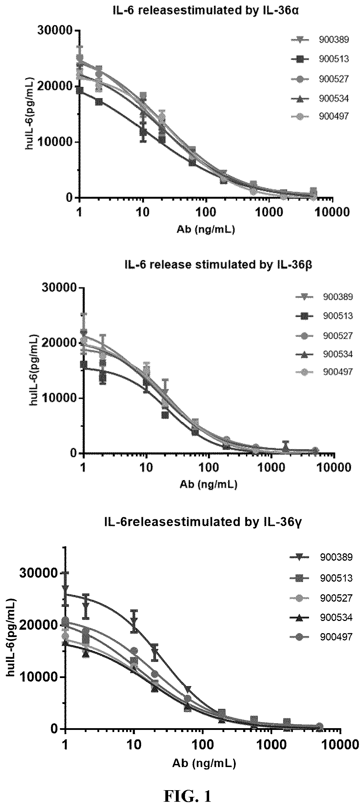

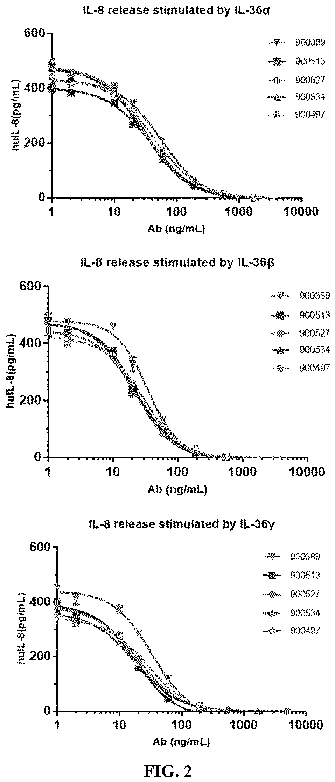

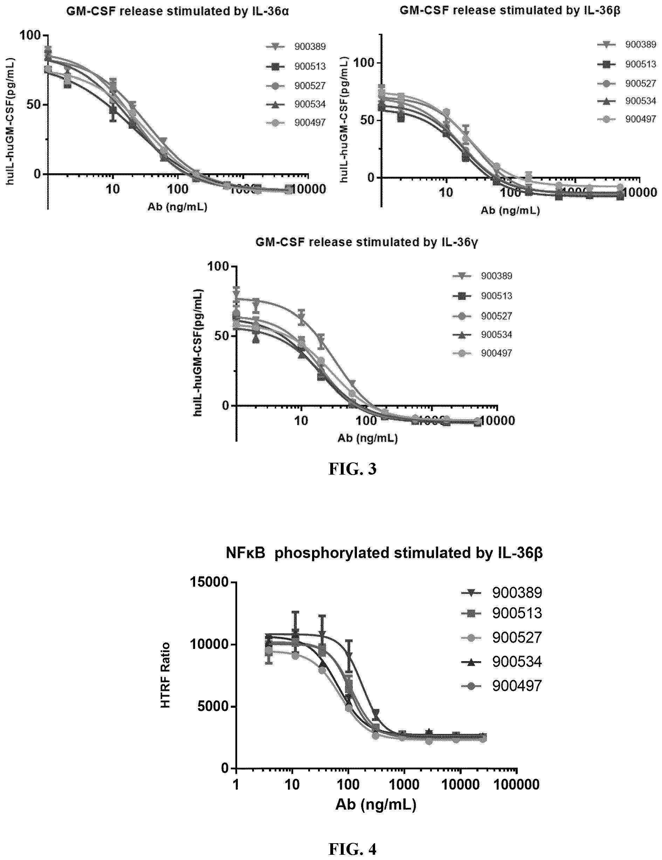

The results were shown as in FIG. 1 to FIG. 4 and Table 4. The humanized antibodies 900513, 900527, and 900534 showed a significant blocking effect on the function of human stimulating factors IL-36α, IL-36β and IL-36γ, such that the secretion of cytokines IL-6, IL-8 and GM-CSF and the phosphorylation of NF-κB could be significantly blocked.

### Example 4 Non-Specific Binding Experiment (SPR) of Chimeric Antibodies and Humanized Antibodies

In this test, the SPR method was used to determine the non-specific adsorption effect between antibodies and non-target molecules, and the instrument and equipment as well as reagent materials used in the test were shown as in Table 5 and Table 6.

**Table 5 Instruments and equipment**

| Designatio n | Factory | Model | No. |
|---|---|---|---|
| Biacore | GE | Biacore 8K | 1305 |

**Table 6 Reagents and materials**

| Designation | Manufacturer/So urce | Cat. No. | Batch No. |
|---|---|---|---|
| Series S Sensor Chip CM5 | GE | BR-1005-30 | 10253038 |
| Egg-derived lysozyme solution | Sigma | L3790 | SLBM3565V |
| Soybean-derived trypsin inhibitor type 1-S | Sigma | T-2327 | SLBJ6832V |
| Polyclonal rabbit anti-lysozyme | ABcam | Ab391 | GR215714-1 |
| Anti-trypsin inhibitor antibodies | LifeSpan Biosciences | LS-C76609 | 26978 |

The Series S Sensor Chip CM5 (GE, #BR-1005-30) was equilibrated for 20-30 min at room temperature and fitted into the Biacore 8K (GE) instrument. The egg-derived lysozyme solution (Sigma, #L3790) and the soybean-derived trypsin inhibitor type 1-S (Sigma, #T-2327) were immobilized to the CMS chip using an amino conjugation kit (GE, #BR-1000-50). HBS-EP(1X) (GE, #BR-1006-69) was used as the loading buffer and 4 equilibrium cycles were set. The polyclonal rabbit anti-lysozyme (ABcam, Ab391), the anti-trypsin inhibitor antibody (LifeSpan Biosciences, #LS-C76609), the chimeric antibody, and the humanized antibody were diluted to 1000 nM with the equilibration buffer, respectively; and the flow rate was set to 5 µL/min, loading channels to 1, 1,2 and 3, and Flow Cells to 1 and 2. The binding time was 10 min and the dissociation time was 15 min. At the regeneration flow rate of 50 µL/min, the regeneration was carried out first with 0.85% phosphoric acid solution (ProteOn, 176-2260) for 60 s, and then with 50 mM sodium hydroxide solution for 30 s.

Here, the sample 900389, as an anti-human IL-36R humanized antibody developed by BI, was obtained through independent expression by HUABO who synthesized variable region genes according to the patent. The sample 900497 was an anti-IL-36R chimeric antibody constructed through hybridoma screening by HUABO.

The results showed that the binding signal of 900389 to the egg-derived lysozyme solution was greater than 20 RU, as shown in Table 7. It was thus believed that the non-specific electrostatic and hydrophobic binding action existed. The binding signal of 900497 to the egg-derived lysozyme solution and the binding signal of 900497 to the soybean-derived trypsin inhibitor type 1-S were both less than 20. It was thus believed that the non-specific electrostatic and hydrophobic binding action did not exist.

**Table 7 Comparison of non-specific binding of samples**

| Designation | Lysozyme (RU) | Trypsin inhibitor (RU) | Deactivated carboxymethyl glucan (RU) | Carboxymethyl glucan (RU) |
|---|---|---|---|---|
| Buffer | 24.0 | 12.9 | 16.2 | 14.0 |
| Polyclonal rabbit anti-lysozyme | 9316.9 | 32.2 | 34.3 | 27.7 |
| Anti-trypsin inhibitor antibodies | 65.1 | 6259.1 | 36.8 | 22.5 |
| 900389 | 55.0 | 27.5 | 16.3 | 14.2 |
| 900497 | 24.3 | 16.7 | 16.4 | 14.4 |

| | | | | |
|---|---|---|---|---|
| Note: It was generally believed that after the influence of buffer was deducted, the response value below 20 RU indicated a weak interaction which was ignorable, the response value greater than 20 RU indicated a significant interaction; and the response value above 100 RU indicated a strong interaction. | | | | |

### Example 5 Cell binding and function experiment of antibodies of stable cell lines

The BI control sequence 900389, the humanized sequence 900527 screened by HUABO, and TNP IgG1 (Fc silence) 900543 were inserted into HUABO GS vectors to construct recombinant expression plasmids for transfecting CHO-K1 cells, respectively, thereby constructing stable cell lines. The stable cell line constructed from HUABO 900527 has a protein number of HB0034, and the screened monoclonal cell lines were optimized by the upstream process to achieve the expression level of greater than 4 g/L. The antibodies expressed by stable cell lines were purified and then tested by flow cytometry binding experiments and functional experiments. The sample 900543 was a TNP IgG1 (Fc silence) negative control antibody.

### 5.1 Flow cytometry binding experiment for stable cell line antibodies

The binding activity of the stable cell line antibodies to human IL-36R and monkey IL-36R was detected, and the cell lines for flow cytometry detection were CHO-K1 cell lines that were constructed internally by HUABO and expressed human IL-36R and monkey IL-36R.

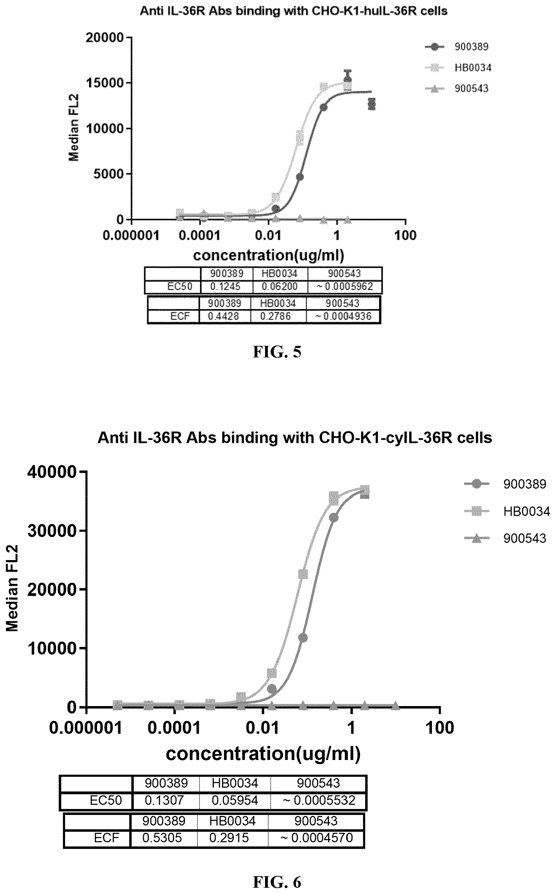

The detection results were shown as in Table 8 and FIG. 5 and FIG. 6.

**Table 8 Detection of binding activity of antibodies to human IL-36R and monkey IL-36R**

| Antibody name | Binding activity to human IL-36R EC50 (ug/mL) | Binding activity to human IL-36R EC90 (ug/mL) | Binding activity to monkey IL-36R EC50 (ug/mL) | Binding activity to monkey IL-36R EC90 (ug/mL) |
|---|---|---|---|---|
| 900389 | 0.1245 | 0.4428 | 0.1307 | 0.5305 |
| HB0034 | 0.062 | 0.2786 | 0.05954 | 0.2915 |
| 900543 | No binding | No binding | No binding | No binding |

The results showed that HUABO monoclonal antibody HB0034 showed a high binding activity to both human IL-36R and monkey IL-36R. Compared with the positive control antibody 900389, the antibody of the present application had lower EC50 value and showed stronger binding activity to human IL-36R.

### 5.2 Experiments on detection of functional inhibition effect of stable cell line antibodies on huII,-36 stimulating factor

### 5.2.1 Functional inhibition effect of antibodies on huIL-36 stimulating factor in NCI/ADR-RES cell s

The human ovarian cancer cell line NCI/ADR-RES was added to a 96-well cell culture plate at 100 µL per well (4.5×10⁴ cells per well), and incubated overnight at 37°C with 5% CO₂; a series of samples to be tested was diluted and then added at 50 µL/well, incubated for 15 min at 37°C with 5% CO₂; the ligand huIL-36β (40 ng/mL) was added at 50 µL/well, mixed thoroughly, and cultured for 18-24 h at 37°C with 5% CO₂. The cell culture supernatant was obtained through centrifuging, and tested in huIL-6 with the HTRF kit (Cat. #: 62HIL06PEG, CISBIO).

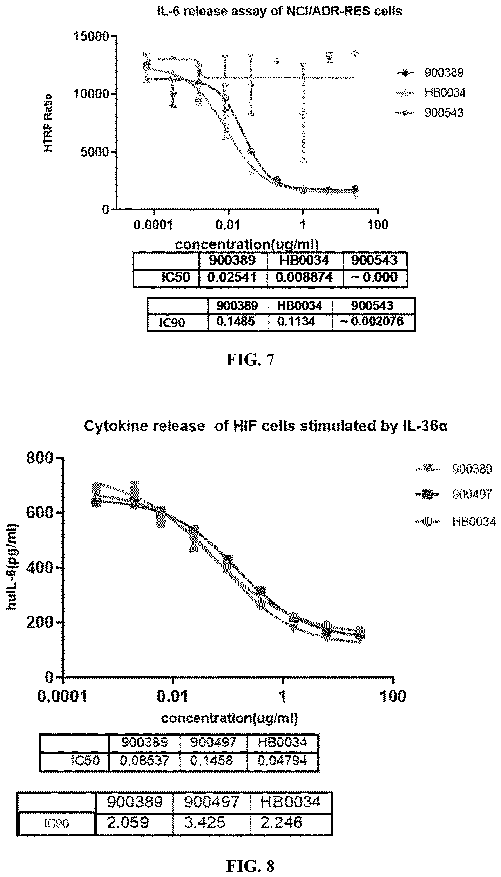

The results were shown as in Table 9 and FIG. 7.

**Table 9 Functional inhibition effects of antibodies against huIL-36 stimulating factors in NCI/ADR-RES cells**

| Antibody name | HuIL-6 detected in NCI/ADR-RES under IL-36β stimulation IC50 (ug/mL) | HuIL-6 detected in NCI/ADR-RES under IL-36β stimulation IC90 (ug/mL) |
|---|---|---|
| 900389 | 0.02541 | 0.1485 |
| HB0034 | 0.008874 | 0.1134 |
| 900543 | No effect | No effect |

The results showed that the humanized antibody HB0034 of the stable cell line had a significant blocking effect on the function of the human stimulating factor IL-36β, and could significantly block the secretion of cytokine IL-6. Compared with the positive control antibody 900389, the antibody of the present application had a lower IC50 value and a stronger blocking effect.

### 5.2.2 Functional inhibition effect of antibodies on huIL-36 stimulating factor in HIF cells

Human intestinal fibroblasts (HIF) cells were prepared into a 4.5E5/ml cell suspension with a Serum Starvd medium, added to a 96-well plate at 100 ul (45,000 cells) per well, and incubated overnight. The series of antibodies was diluted, added at 50 µl/well, and incubated for 15 min at 37°C. IL-36α was diluted to 1000 ng/ml, added at 50 µl per well, and mixed thoroughly. The mixture was incubated for 4 h at 37°C with 5% CO₂, and centrifuged to collect a supernatant, in which the content of human IL-6 was detected. IL-36β was diluted to 80 ng/m, added at 50 µl per well, and mixed thoroughly. The mixture was incubated for 4 h at 37°C with 5% CO₂, and centrifuged to collect a supernatant, in which the content of human IL-6 was detected. IL-36β was diluted to 40ng/m, added at 50 µl per well, and mixed thoroughly. The mixture was incubated for 20 min at 37°C with 5% CO₂, and the content of pNFκB was detected using the kit.

**Table 10 Functional inhibition effects of antibodies against huIL-36 stimulating factor in HIF cells**

| Name of antibody | HuIL-6 detected in HIF under IL-36α stimulation IC50 (ug/mL) | HuIL-6 detected in HIF under IL-36α stimulation IC90 (ug/mL) |
|---|---|---|
| 900389 | 0.08537 | 2.059 |
| 900497 | 0.1458 | 3.425 |
| HB0034 | 0.04794 | 2.246 |

**Table 11 Functional inhibition effects of antibodies against huIL-36 stimulating factor in HIF cells**

| Name of antibody | HuIL-6 detected in HIF under IL-36β stimulation IC50 (ug/mL) | HuIL-6 detected in HIF under IL-36β stimulation IC90 (ug/mL) |
|---|---|---|
| 900389 | 0.07276 | 1.386 |
| 900497 | 0.08373 | 3.644 |
| HB0034 | 0.05458 | 2.138 |

**Table 12 Functional inhibition effects of antibodies against huIL-36 stimulating factor in HIF cells**

| Name of antibody | pNFκB detected in HIF under IL-36β stimulation IC50 (ug/mL) | pNFκB detected in HIF under IL-36β stimulation IC90 (ug/mL) |
|---|---|---|
| 900389 | 0.1670 | 0.4188 |
| 900497 | 0.1002 | 0.7757 |
| HB0034 | 0.1049 | 0.6863 |

The results showed that the humanized antibody HB0034 of the stable cell line had a significant blocking effect on the function of human stimulating factors IL-36α and IL-36β, and could significantly block the secretion of cytokine IL-6 and the phosphorylation of NF-κB. Compared with the positive control antibody 900389, the antibody of the present application had a lower IC50 value and a stronger blocking effect.

### Example 6 Sequence Design of Heavy and Light chains of IL17A-IL36R Bifunctional Antibodies and Preparation of Bispecific Antibodies

Based on the anti-IL17A antibody previously developed by the applicants and the anti-IL36R antibody obtained in the present application, IL17A-IL36R bifunctional antibodies were constructed. In this example, the anti-IL36R antibody 900527 prepared in Example 2 was used as an example for construction of the bi-antibody. The method was as follows.

### 6.1 Sequence design

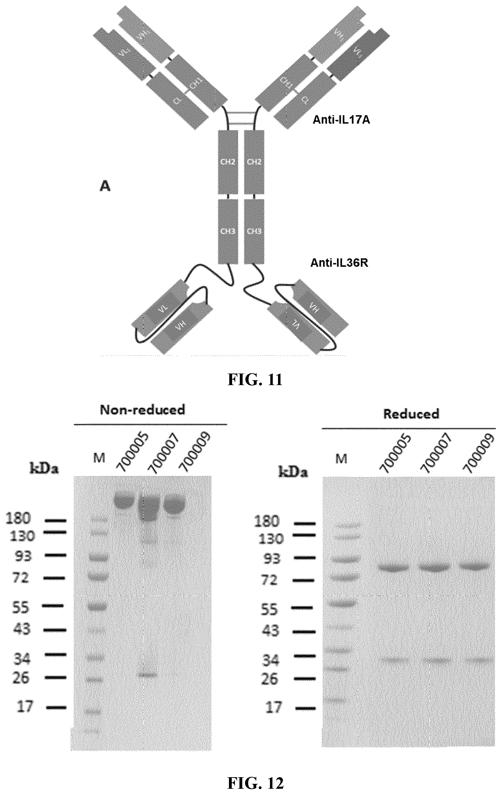

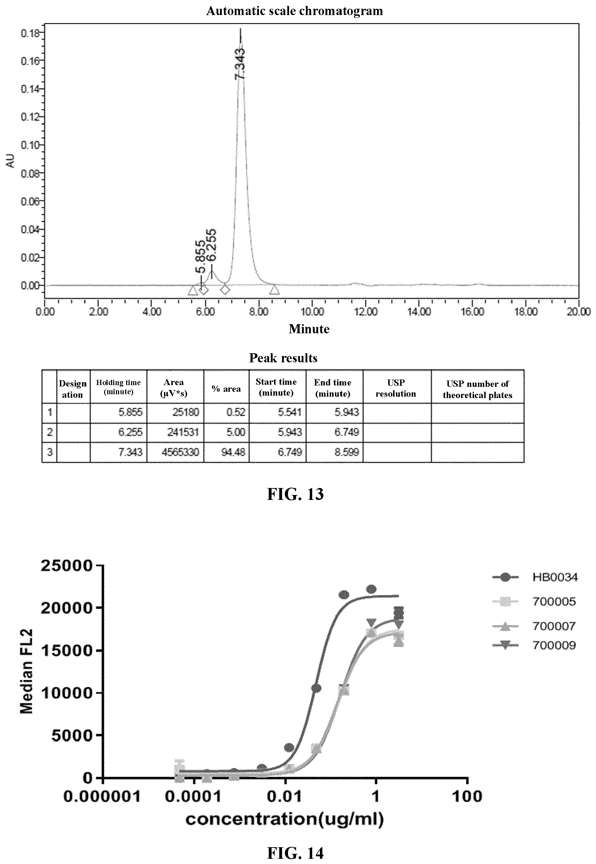

The structure format of the IL17A-IL36R bispecific antibody of the present application was the Morrison format (IgG-scFv), i.e., linking the C-termini of two heavy chains in one IgG antibody to the scFv fragment of another antibody, with a configuration as shown in FIG. 11 and the main component design of heavy and light chains as shown in FIG. 13

**Table 13**

| Bispecific antibody No. | Heavy chain | | | Light chain variable region |
|---|---|---|---|---|
| | Heavy-chain variable region of IgG moiety | Linker fragment | scFv moiety | |
| 700006 | IL17-HC5-1D4 | Linker2 | (900527-VL-Q100C) -Linkerl-(900527-VH-G44C) | IL17-LC5-1C5 |
| 700008 | IL17-HC5-1D4 | Linker2 | (900527-VL-Q100C) - Linker2- (900527-VH-G44C) | IL17-LC5-1C5 |
| 700010 | IL17-HC5-1D4 | Linker2 | (900527-VL-Q100C) - Linker3- (900527-VH-G44C) | IL17-LC5-1C5 |
| 700005 | IL17-HC5-1D4 | Linker2 | (900527-VL) -Linker 1-(900527-VH) | IL17-LC5-1C5 |
| 700007 | IL17-HC5-1D4 | Linker2 | (900527-VL) -Linker2-(900527-VH) | IL17-LC5-1C5 |
| 700009 | IL17-HC5-1D4 | Linker2 | (900527-VL) -Linker3-(900527-VH) | IL17-LC5-1C5 |

| | | | | |
|---|---|---|---|---|
| Note 1: IL17-HC5-1D4 was derived from the patented anti-IL17 humanized monoclonal antibody (CN107522783B) of Huabo Biopharm (Shanghai) Co., Ltd., and had the same sequence as SEQ ID NO: 5 for the patented antibody in CN107522783B; and IL17-LC5-1C5 was derived from the patented anti-IL17 humanized monoclonal antibody (CN107522783B) of Huabo Biopharm (Shanghai) Co., Ltd., and had the same sequence as SEQ ID NO: 6 for the patented antibody in CN107522783B. Note 2: 900527 was derived from an anti-IL36R humanized monoclonal sequence prepared in Example 2 of the present application. 900527-VL referred to the light-chain variable region of 900527, and 900527-VH referred to the heavy-chain variable region of 900527. Note 3: Q100C referred to mutation of an amino acid Q at position 100 in the light-chain variable region into C according to the Kabat antibody numbering scheme; and R44C or G44C referred to mutation of an amino acid G at position 44 in the heavy-chain variable region into C according to the Kabat antibody numbering scheme. A disulfide bond might be formed after these two amino acid mutations, which enhanced the stability of the scFv region, and increased the druggability of the bi-antibodies. Note 4: Amino acid sequence of Linker 1: (G4S)3 Amino acid sequence of Linker 2: (G4S)4 Amino acid sequence of Linker 3: (G4S)5 | | | | |

### 1.2 Sequences of bispecific antibody

The heavy chain sequences of the bispecific antibody can be found in Table 14 below.

**Table 14**

| Protein number | Heavy chain sequence |
|---|---|
| 00005 | QVQLVQSGSELKKPGASVKVSCKASEYIFTNYGMNWVKQAPGQGFEW MGWIDTNTGEPTYAQGFTGRFVFSLDTSVSTAYLQISSLKAEDTATYYCA NYGWGYFDYWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKRVEPKSCDKTHTCPPCPAPEAAGGPSVFLFPPKPKDTLMIS RTPEVTCVVVDVSHEDPEVKFNWYVDGVEVHNAKTKPREEQYNSTYRVVS VLTVLHQDWLNGKEYKCKVSNKALPAPLEKTISKAKGQPREPQVYTLPPSRE EMTKNQVSLICLVKGFYPSDIAVEWESNGQPENNYKTTPPVLDSDGSFFLYSK LTVDKSRWQQGNVFSCSVMHEALHNHYTQKSLSLSPGGGGGSGGGGSGGG GSGGGGSQIVLTQSPGTLSLSPGERATLSCRASSSVSSSYLHWYQQKPGQ APRLWIYSTSSRATGIPDRFSGSGSGTDFTLTISRLEPEDFATYYCQQFQSS PLTFGQGTKLEIKGGGGSGGGGSGGGGSQVQLVQSGAEVKKPGASVKVS CKASGYTFTSSWMHWAKQAPGQGLEWIGEIHPNSAKTNYNQKFQGRVT MTRDTSISTAYMELSRLRSDDTAVYYCARVDYGKPWFAYWGQGTLVTVS S (SEQ ID NO:13) |
| 00006 | QVQLVQSGSELKKPGASVKVSCKASEYIFTNYGMNWVKQAPGQGFEW MGWIDTNTGEPTYAQGFTGRFVFSLDTSVSTAYLQISSLKAEDTATYYCA NYGWGYFDYWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKRVEPKSCDKTHTCPPCPAPEAAGGPSVFLFPPKPKDTLMIS RTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRE EMTKNQVSLTCLVKGFYPSDIAVEWESNGQPENNYKTTPPVLDSDGSFFLYSK |
| | LTVDKSRWQQGNVFSCSVMHEALHNHYTQKSLSLSPGGGGGSGGGGSGGG GSGGGGSQIVLTQSPGTLSLSPGERATLSCRASSSVSSSYLHWYQQKPGQ APRLWIYSTSSRATGIPDRFSGSGSGTDFTLTISRLEPEDFATYYCQQFQSS PLTFGCGTKLEIKGGGGSGGGGSGGGGSQVQLVQSGAEVKKPGASVKVS CKASGYTFTSSWMHWAKQAPGQCLEWIGEIHPNSAKTNYNQKFQGRVT MTRDTSISTAYMELSRLRSDDTAVYYCARVDYGKPWFAYWGQGTLVTVS S (SEQ ID NO: 14) |
| 00007 | QVQLVQSGSELKKPGASVKVSCKASEYIFTNYGMNWVKQAPGQGFEW MGWIDTNTGEPTYAQGFTGRFVFSLDTSVSTAYLQISSLKAEDTATYYCA NYGWGYFDYWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKRVEPKSCDKTHTCPPCPAPEAAGGPSVFLFPPKPKDTLMIS RTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRE EMTKNQVSLTCLVKGFYPSDIAVEWESNGQPENNYKTTPPVLDSDGSFFLYSK LTVDKSRWQQGNVFSCSVMHEALHNHYTQKSLSLSPGGGGGSGGGGSGGG GSGGGGSQIVLTQSPGTLSLSPGERATLSCRASSSVSSSYLHWYQQKPGQ APRLWIYSTSSRATGIPDRFSGSGSGTDFTLTISRLEPEDFATYYCQQFQSS PLTFGQGTKLEIKGGGGSGGGGSGGGGSGGGGSQVQLVQSGAEVKKPGAS VKVSCKASGYTFTSSWMHWAKQAPGQGLEWIGEIHPNSAKTNYNQKFQ GRVTMTRDTSISTAYMELSRLRSDDTAVYYCRVDYGKPWFAYWGQGTLV TVSS (SEQ ID NO:15) |
| 00008 | QVQLVQSGSELKKPGASVKVSCKASEYIFTNYGMNWVKQAPGQGFEW MGWIDTNTGEPTYAQGFTGRFVFSLDTSVSTAYLQISSLKAEDTATYYCA NYGWOYRDYWGQGTTVTVSSASTKGPSVFPLAPSSKSTSGGTAALGCLVK DYFPEPVTVSWNSGALTSGVHIFPAVLQSSGLYSLSSVVTVPSSSLGTQTYICN VNHKPSNTKVDKKVEPKSCDKTHTCPPCPAPEAAGGPSVFLFPPKPKDTLMIS RTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRE EMTKNQVSLTCLVKGFYPSDIAVEWESNGQPENNYKTTPPVLDSDGSFFLYSK LTVDKSRWQQGNVFSCSVMHEALHNHYTQKSLSLSPGGGGGSGGGGSGGG GSGGGGSQIVLTQSPGTLSLSPGERATLSCRASSSVSSSYLHWYQOKPGQ APRLWIYSTSSRATGIPDRFSGSGSGTDFTLTISRLEPEDFATYYCQQFQSS PLTFGCGTKLEIKGGGGSGGGGSGGGGSGGGGSQVQLVQSGAEVKKPGAS VKVSCKASGYTFTSSWMHWAKQAPGQCLEWIGEIHPNSAKTNYNQKFQ GRVTMTRDTSISTAYMELSRLRSDDTAVYYCARVDYCKPWFAYWGQGTL VTVSS (SEQ ID NO: 16) |
| 00009 | QVQLVQSGSELKKPGASVKVSCKASEYIFTNYGMNWVKQAPGQGFEW MGWIDTNTGEPTYAQGFTGRFVFSLDTSVSTAYLQISSLKAEDTATYYCA NYGWGYFDYWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNIKVDKKVEPKSCDKTHTCPPCPAPEAAGGPSVFLFPPKPKDTLMIS RTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRE EMTKNQVSLTCLVKGFYPSDIAVEWESNGQPENNYKTTPPVLDSDGSFFLYSK LTVDKSRWQQGNVFSCSVMHEALHNHYTQKSLSLSPGGGGGSGGGGSGGG GSGGGGSQIVLTQSPGTLSLSPGERATLSCRASSSVSSSYLHWYQQKPGQ |
| | APRLWIYSTSSRATGIPDRFSGSGSGTDFTLTISRLEPEDFATYYCQQFQSS PLTFGQGTKLEIKGGGGSGGGGSGGGGSGGGGSGGGGSQVQLVQSGAEVK KPGASVKVSCKASGYTFTSSWMHWAKQAPGQGLEWIGEIHPNSAKTNY NQKFQGRVTMTRDTSISTAYMELSRLRSDDTAVYYCARVDYGKPWFAYW GQGTLVTVSS (SEQ ID NO: 17) |
| 00010 | QVQLVQSGSELKKPGASVKVSCKASEYIFTNYGMNWVKQAPGQGFEW MGWIDTNTGEPTYAQGFTGRFVFSLDTSVSTAYLQISSLKAEDTATYYCA NYGWGYFDYWGQGTTVTVSSASTKGPSVFPLAPSSKSTSGGTAALGCLVK DYFPEPVTVSWNSGALTSGVHTFPAVLQSSGLYSLSSVVTVPSSSLGTQTYICN VNHKPSNTKVDKRVEPKSCDKTHTCPPCPAPEAAGGPSVFLFPPKPKDTLMIS RTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRE EMTKNQVSLTCLVKGFYPSDIAVEWESNGQPENNYKTTPPVLDSDGSFFLYSK LTVDKSRWQQGNVFSCSVMHEALHNHYTQKSLSLSPGGGGGSGGGGSGGG GSGGGGSQIVLTQSPGTLSLSPGERATLSCRASSSVSSSYLHWYQQKPGQ APRLWIYSTSSRATGIPDRFSGSGSGTDFTLTISRLEPEDFATYYCQQFQSS PLTFGCGTKLEIKGGGGSGGGGSGGGGSGGGGSGGGGSQVQLVQSGAEVK KPGASVKVSCKASGYTFTSSWMHWAKQAPGQCLEWIGEIHPNSAKTNY NQKFQGRVTMTRDTSISTAYMELSRLRSDDTAVYYCARVDYGKPWFAYW GQGTLVTVSS (SEQ ID NO: 18) |

All light chain sequences of the bispecific antibody were identical, with the light chain sequences as follows:

DVVMTQTPPSLPVNPGEPASISCRSSQSLVHSNGYTYLHWYLQKPGQSPQLLIYKV
SNHLSGVPDRFSGSGSGTDFTLKISWVEAEDVGVYFCSQSTHVPYTFGGGTKLEIKR
TVAAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO: 19)

Here, the heavy-chain variable region of IL17A scFv had an amino acid sequence as follows:

QvQLVQSGSELKKPGASVKVSCKASEYIFTNYGMNWVKQAPGQGFEWMGWIDTNTGE
PTYAQGFTGRFVFSLDTSVSTAYLQISSLKAEDTATYYCANYGWGYFDYWGQGTTVTVS
S(SEQ ID NO: 26）

It included the following three complementary determining regions (CDRs):
CDR1 (EYIFTNY) as set forth in SEQ ID NO: 20,
CDR2 (DTNTGE) as set forth in SEQ ID NO: 21, and
CDR3 (ANYGWGYFDY) as set forth in SEQ ID NO: 22.

The heavy-chain variable region of IL17A scFv had an amino acid sequence as follows:

DVVMTQTPPSLPVNPGEPASISCRSSQSLVHSNGYTYLHWYLQKPGQSPQLLIYKVSNHL
SGVPDRFSGSGSGTDFTLKISWVEAEDVGVYFCSQSTHVPYTFGGGTKLEIK (SEQ ID NO:
27)

It included the following three complementary determining regions (CDRs):
CDR1' (QSLVHSNGYTY) as set forth in SEQ ID NO: 23,
CDR2' (KVS) as set forth in SEQ ID NO: 24, and
CDR3' (SQSTHVPYT) as set forth in SEQ ID NO: 25.

Mammalian cells such as HEK293 were transfected with the above heavy and light chains using a transient or stable transfection protocol. A cell culture solution was centrifuged at high speed and then purified with an affinity packing Protein A to prepare the IL17A/IL36R bispecific antibodies.

### Example 7 Expression and Purification of Bispecific Antibodies

The expression vectors obtained from Example 6 were amplified by means of *Escherichia coli,* and a sufficient amount of plasmids were prepared using an endotoxin-free plasmid extraction kit (Tiangen Biotech (Beijing) Co., Ltd., #DP117) for transient transfection to express the bispecific antibody. CHO-S cells (ThermoFisher, #R80007) were used as host cells for expression. The two prepared heavy-chain vectors, respectively with the light-chain vectors, were mixed with polyetherimide (PEI, Polysciences, #24765-1) to form liposome complexes, which were transfected into CHO-S cells and incubated in an incubator for 5-7 days. The cell culture supernatant was collected through centrifugation, and purified by the Protein A affinity chromatographic column to obtain a series of bispecific antibodies, and the protein aggregation was further determined by the sieve chromatography.

The expression, purification and detection of the bispecific antibodies can be found in FIG. 12, FIG. 13 and Table 15, and the test results show that 700007 and 700009 have good purity. The reduced and non-reduced SDS-PAGE patterns didn't show significant degradation of three bispecific antibodies, indicating good protein stability.

**Table 15 SEC purity data of IL17A-IL36R bispecific antibodies**

| Antibody | 700005 | 700007 | 700009 |
|---|---|---|---|
| SEC Purity (%) | 86.37 | 91.9 | 94.48 |

### Example 8 Detection of Thermostability of IL17A-IL36R Bispecific Antibody

The experimental procedures for the thermostability of the IL17A-IL36R bispecific antibodies were as follows: detecting the melting temperature (Tm) and aggregation temperature (Tagg) of the IL17A-IL36R bispecific antibodies 70005, 700007 and 700009 by a protein stability analyzer (UNcle, UNCHAINED LABS, US), with the temperature rise range of 25°C to 95°C and the heating rate of 0.3°C for Tm and Tagg.

The detection results of the thermostability of the bispecific antibodies in the form of IL17AIL36R can be found in Table 16 below, and the results show that, in the thermostability data, the Tm values of the bispecific antibodies 70005, 700007 and 700009 in the form of IL17A-IL36R were all in the range of 65°C to 70°C, indicating good thermostability for these three bispecific antibodies.

**Table 16 Measurement of thermal stability data Tm and Tagg values of bispecific antibodies in the form of IL17A-IL36R**

| Antibody | Tm value (°C) | Tagg value (°C) |
|---|---|---|
| 700005 | 67.8 | 61.6 |
| 700007 | 67.7 | 60.7 |
| 700009 | 68.3 | 61.8 |

### Example 9 Detection of Thermostability of IL17A-IL36R Bispecific Antibody

In this test, the binding kinetics and affinity of the IL17A-IL36R bispecific antibodies, IL17A parental monoclonal antibody HB0017 and IL36R parental monoclonal antibody HB0034, to antigens were determined using the SPR method.

The test method was as follows.

The Anti-Human Capture-CM5 chip (GE, #BR-1005-30) was prepared according to the method for conjugating the Human Antibody Capture Kit (GE, #BR-1008-39) to the Amino Conjugation Kit (GE, BR-1000-50). The chip was equilibrated for 20-30 min at room temperature and loaded into the Biacore 8K instrument; the antigens and antibodies were diluted to experimental working concentrations with the equilibration buffer; the antigens were diluted to 50 nM with the equilibrium buffer, and then diluted 3-fold for 7 concentration gradients, and set 2 zero concentrations (i.e., the equilibrium buffer) and one repeat concentration (generally repeating at the minimal concentration); following the order of antibodies, antigens and regeneration, 10 antigen concentrations (2 zero concentrations, 7 gradient concentrations and 1 repeat concentration) were experimentally analyzed in a cyclical manner, and the antigen injection flow rate was 30 µL/min, the binding time was 120 seconds, and the dissociation time was 600 seconds; and after the analysis was completed, the corresponding analysis program was selected to analyze the data, no obvious reference binding was determined, and Kinetics, 1: 1 binding model was selected to fit the data to obtain the kinetics related parameters Ka, Kd and KD values of the bispecific antibodies and the monoclonal-antibody parent antibodies.

The test results can be found in Table 17. The results show that, for the IL17A-IL36R bispecific antibodies, the affinity of bispecific antibodies 70005, 700007 and 700009 to IL17Ais comparable to that of the parent HB0017, and the affinity of the bispecific antibodies 700005, 700007 and 700009 to IL36R is expected to be comparable to that of the parent HB0034.

**Table 17 Detection of affinity of IL17A-IL36R bispecific antibodies to antigens**

| Ligand | Analyte | ka (1/Ms) | kd (1/s) | KD (M) |
|---|---|---|---|---|
| HB0017 | 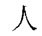 IL17A | 7.98E+06 | 7.99E-05 | 1.00E-11 |
| 700005 | 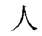 IL17A | 6.77E+06 | 4.13E-05 | 6.10E-12 |
| 700007 | 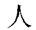 IL17A | 7.06E+06 | 4.33E-05 | 6.13E-12 |
| 700009 | 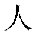 IL17A | 7.17E+06 | 5.71E-05 | 7.96E-12 |
| HB0034 | 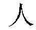 IL36R | 1.07E+06 | 1.50E-04 | 1.41E-10 |
| 700005 | 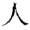 IL36R | 2.05E+05 | 7.20E-05 | 3.50E-10 |

### Example 10 Detection of Thermostability of IL17A-IL36R Bispecific Antibody

### 10.1 Determination of binding activity of IL17A-IL36R bispecific antibodies to IL36R receptors on cell membranes

The experimental procedures for detecting the binding activity of IL17A-IL36R bispecific antibodies to IL36R receptors on cell membranes were as follows. CHO-K1-huIL-36R-5E10/2F10 cells overexpressing the IL36R receptors were counted, resuspended to 1×10E6/mL, and plated to a 96-well white bottom plate at 2 µl per well. Then, the bispecific antibodies and the parental monoclonal antibody HB0034 were diluted to 100 µg/ml with the PBS solution, and then diluted 4-fold for 11 gradients. The diluted antibodies were added to the 96-well plate and incubated for 30 min at room temperature. Then, 100 µl of FACS solution was added per well for rinsing; the cells were resuspended and centrifuged for 3 min at 300 g; the supernatant was discarded; and rinsing was repeated once. After the rinsing was completed, 20 µl of PE-GAH solution diluted at 1:200 was added per well, and incubation was conducted for 15 min at room temperature. After the incubation, rinsing was conducted twice with the FACS solution, and after the rinsing was completed, reading was conducted to detect the signal intensity.

The detection results of the IL17A-IL36R bispecific antibodies can be found in FIG. 14 and Table 18. The results show that the binding activity of the bispecific antibodies 700005, 700007 and 700009 to IL36R receptors on cell membranes is comparable to that of the IL36R parent monoclonal antibody HB0034.

**Table 18 Binding activity of IL17A-IL36R bispecific antibodies to IL36R receptors on cell membranes**

| Antibody | HB0034 | 700005 | 700007 | 700009 |
|---|---|---|---|---|
| EC50 (nM) | 0.32 | 0.747 | 0.693 | 0.799 |

### 10.2 Determination of binding activity of IL17A-IL36R bispecific antibodies to IL17A

Human IL-17A was prepared with CBS into a 1 µg/ml coating solution, and added to the ELISA plate at 100µL/well for coating for more than 12 hours at 2-8°C; then, the plate residue was discarded; 3% milk was added at 200 µL per well; and the plate was closed at room temperature for 1.5 hours. Not less than 200 µL of PBST was added to each well for washing 3 times; the bispecific antibodies and the HB0017 parental monoclonal antibody were diluted to 100 µg/ml, then diluted 5-fold over 10 gradients, and added to the ELISA plate at 100 µL/well. Incubation was conducted for 1 h at room temperature; not less than 200 µL of PBST was added per well for washing 4 times; then, 3% milk-PBST was added to dilute HRP-conjugated goat anti-mouse IgG Fc (purchased from Jacksot) 25,000-fold, which was then added at 100 µL/well. After 1 h of incubation at room temperature, not less than 200 µL of PBST was added per well for washing 6 times, and the plate was dried by patting. A TMB chromogenic solution was added at 100 µL per well. After 5 min of reaction at room temperature, 2M H2SO4 was added 50 µL/well to terminate the reaction. The ELISA plate with the reaction terminated was placed on a microplate reader, and the absorbance OD450 value was read at a wavelength of 450 nm.

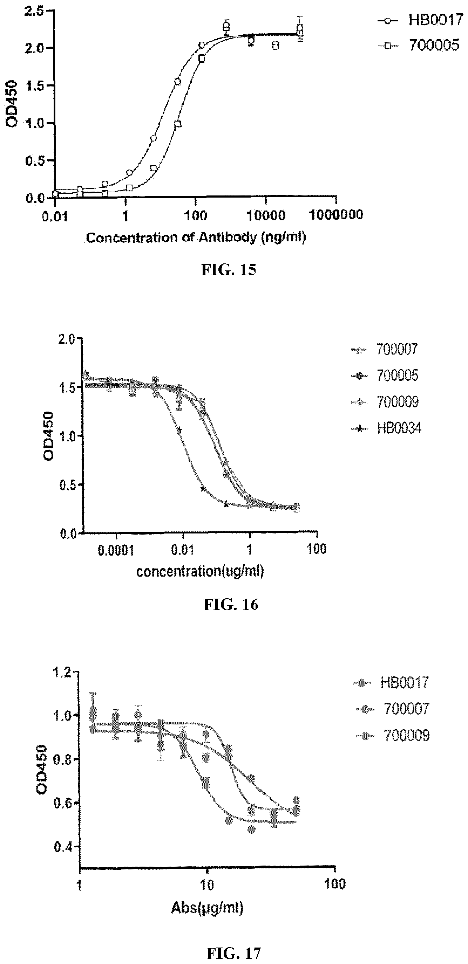

The detection results of the IL17A-IL36R bispecific antibodies can be found in FIG. 15 and Table 19. The binding activity of the bispecific antibodies 700005, 700007, and 700009 to the IL-17A recombinant protein was expected to be comparable to that of the IL17A parent monoclonal antibody HB0017.

**Table 19 Determination of binding activity of IL17A-IL36R bispecific antibodies to IL17A**

| Antibody | HB0017 | 700005 |
|---|---|---|
| EC50(nM) | 0.0858 | 0.1803 |

### Example 11 Detection of Thermostability of IL17A-IL36R Bispecific Antibody

### 11.1 Functional inhibition effects of IL17A-IL36R bispecific antibodies against IL36R

The experimental procedures for detecting the functional inhibition effects of the IL17A-IL36R bispecific antibodies against IL36R were as follows. NCI/ADR-RES cells were prepared with a (Ficoll-Paque, GE) was added to a SepMate-50^{™} (StemCell) isolation tube; the diluted whole blood was slowly added along the tube wall; centrifugation was conducted for 20 min at 1200 g at room temperature; the upper liquid (a PBMC layer) was poured from the isolation tube into a new 50 ml centrifuge tube, and washed twice with 2% FBS/PBS.
2) Single-core isolation: The PBMC was resuspended with the isolation solution; a concentrated mixture (Enrichment Cocktail) was added according to the kit instructions (STEMCELL Cat#: 19058); the resultant was incubated for 10 min at 4°C; magnetic beads was added at 50 µL/mL; the resulting mixture was incubated for 10 min at 4°C; and the magnetic beads were removed in a magnetic field.
3) Induced differentiation of moDC: Monocytes obtained in 2) were counted in a 1640 medium and resuspended to 5E5 cells/mL; and GM-CSF with a final concentration of 10 ng/ml and IL-4 with a final concentration of 10 ng/ml were added for incubation for 6 days.
4) IL-36 stimulation: moDCs collected in 3) were plated at a density of 3E4 per well; IL-36α and the gradient-diluted antibody (HB0043) were added, with human IgG1 (TNP) as a negative control; incubation was conducted for 24 h; and then, the IL-6 level were detected with the Human IL-6 ELISA kit.

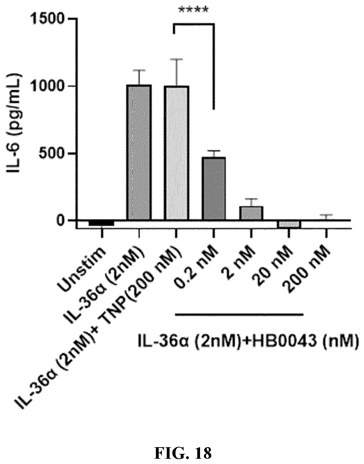

The experimental results can be found in FIG. 18, and show that the bispecific antibodies can block IL-6 secretion by IL-36α-activated moDCs and have the inhibitory effect increasing with increasing concentrations.

All documents mentioned in the present application are hereby incorporated by reference in their entirety, just as each document is cited separately as a reference. In addition, it should be understood that various modifications and changes may be made by those skilled in the art after reading the above teachings of the present application and these equivalent forms also fall within the scope defined by the claims appended hereto.

## Claims

1. A bispecific antibody targeting IL-17A and IL-36R, having one or more of the following properties:
a) high-affinity binding to IL-36R;
b) high-affinity binding to IL-1 Rrp2;
c) blocking binding of IL-36α, IL-36β, and IL-36γ to IL-36R; and
d) blocking cytokine secretion mediated by IL-36α, IL-36β or IL-36γ.

2. The bispecific antibody according to claim 1, wherein an affinity constant KD(M) of said bispecific antibody for binding to a human IL-36R protein is (0.5-10)×10⁻¹¹.

3. The bispecific antibody according to any one of claims 1 to 2, wherein an affinity constant KD(M) of said bispecific antibody for binding to human IL-1 Rrp2 is (0.5-10)×10⁻¹¹.

4. The bispecific antibody according to any one of claims 1 to 3, wherein said cytokine comprises: IL-6, IL-8, and GM-CSF.

5. The bispecific antibody according to any one of claims 1 to 4, wherein said bispecific antibody is selected from the group consisting of: animal derived antibodies, chimeric antibodies, humanized antibodies, or combinations thereof.

6. The bispecific antibody according to any one of claims 1 to 5, wherein said bispecific antibody is a double-chain antibody or a single-chain antibody.

7. The bispecific antibody according to any one of claims 1 to 6, wherein said bispecific antibody is a monoclonal antibody.

8. The bispecific antibody according to any one of claims 1 to 7, wherein said bispecific antibody is a partially or fully humanized monoclonal antibody.

9. The bispecific antibody according to any one of claims 1 to 8, wherein said bispecific antibody comprises a first targeting moiety capable of specifically binding to an IL-36R protein, wherein said first targeting moiety comprises an isolated antigen-binding protein comprising a heavy-chain variable region VH, which comprises three complementarity determining regions CDR1-3, with said CDR1 as set forth in SEQ ID NO: 3.

10. The bispecific antibody according to claim 9, wherein said CDR2 is as set forth in SEQ ID NO: 4.

11. The bispecific antibody according to any one of claims 9 to 10, wherein said CDR3 is as set forth in SEQ ID NO: 5.

12. The bispecific antibody according to any one of claims 9 to 11, wherein said heavy-chain variable region further comprises a human-derived FR or a murine FR.

13. The bispecific antibody according to any one of claims 9 to 12, wherein said heavy-chain variable region has an amino acid sequence as set forth in SEQ ID NO: 1.

14. The bispecific antibody according to any one of claims 9 to 13, wherein said heavy-chain variable region has an amino acid sequence as set forth in SEQ ID NO: 9.

15. The bispecific antibody according to any one of claims 1 to 14, wherein said bispecific antibody comprises a light-chain variable region VL, which comprises the following three complementary determining regions: CDR1'-3', with said CDR1' as set forth in SEQ ID NO: 6.

16. The bispecific antibody according to claim 15, wherein said CDR2' is as set forth in SEQ ID NO: 7.

17. The bispecific antibody according to any one of claims 15 to 16, wherein said CDR3' is as set forth in SEQ ID NO: 8.

18. The bispecific antibody according to any one of claims 15 to 17, wherein said light-chain variable region further comprises a human-derived FR or a murine FR.

19. The bispecific antibody according to any one of claims 15 to 18, wherein said light-chain variable region has an amino acid sequence as set forth in SEQ ID NO: 2.

20. The bispecific antibody according to any one of claims 15 to 19, wherein said light-chain variable region has an amino acid sequence as set forth in SEQ ID NO: 10, 11 or 12.

21. The bispecific antibody according to any one of claims 1 to 20, wherein said bispecific antibody further comprises a second targeting moiety capable of specifically binding to IL-17A.

22. The bispecific antibody according to any one of claims 1 to 21, wherein said bispecific antibody comprises: (a) an anti-IL-17A antibody; and (b) a single-chain variable region ScFv of an anti-IL-36R antibody linked to said anti-IL-17A antibody.

23. The bispecific antibody according to claim 22, wherein a heavy-chain variable region of said anti-IL-17A antibody comprises the following three complementary determining regions: CDR1-3, with said CDR1 as set forth in SEQ ID NO: 20.

24. The bispecific antibody according to claim 23, wherein said CDR2 is as set forth in SEQ ID NO: 21.

25. The bispecific antibody according to any one of claims 23 to 24, wherein said CDR2 is as set forth in SEQ ID NO: 22.

26. The bispecific antibody according to any one of claims 22 to 25, wherein a light-chain variable region of said anti-IL-17A antibody comprises the following three complementary determining regions: CDR1'-3', with said CDR1' as set forth in SEQ ID NO: 23.

27. The bispecific antibody according to claim 26, wherein said CDR2' is set forth in SEQ ID NO:24.

28. The bispecific antibody according to any one of claims 26 to 27, wherein said CDR3' is as set forth in SEQ ID NO: 25.

29. The bispecific antibody according to any one of claims 22 to 28, wherein in an ScFv of said anti-IL-17A antibody, a heavy-chain variable region is set forth in SEQ ID NO: 26.

30. The bispecific antibody according to any one of claims 22 to 29, wherein in said ScFv of said anti-IL-17A antibody, a light-chain variable region is set forth in SEQ ID NO: 27.

31. The bispecific antibody according to any one of claims 22 to 30, wherein said anti-IL-17A antibody and said ScFv of said anti-IL-36R antibody are linked via a linker sequence.

32. The bispecific antibody according to any one of claims 22 to 31, wherein in said ScFv of said anti-IL-36R antibody, a heavy-chain variable region of said anti-IL-36R antibody has an amino acid sequence as set forth in SEQ ID NO: 1; and a light-chain variable region of said anti-IL-36R antibody has an amino acid sequence as set forth in SEQ ID NO: 2.

33. The bispecific antibody according to any one of claims 22 to 32, wherein in said ScFv of said anti-IL-36R antibody, said heavy-chain variable region of said anti-IL-36R antibody has an amino acid sequence as set forth in SEQ ID NO: 9; and said light-chain variable region of said anti-IL-36R antibody has an amino acid sequence as set forth in SEQ ID NO: 10, 11 or 12.

34. The bispecific antibody according to any one of claims 22 to 33, wherein in said ScFv of said anti-IL-36R antibody, said heavy-chain variable region has an amino acid sequence as set forth in SEQ ID NO: 9; and said light-chain variable region has an amino acid sequence as set forth in SEQ ID NO: 11.

35. The bispecific antibody according to any one of claims 22 to 34, wherein in said ScFv of said anti-IL-36R antibody, an amino acid residue G at position 44 is mutated into C in said amino acid sequence of said heavy-chain variable region of said anti-IL-36R antibody, based on SEQ ID NO: 9.

36. The bispecific antibody according to any one of claims 22 to 35 wherein in said ScFv of said anti-IL-36R antibody, an amino acid residue Q at position 101 is mutated into C in said amino acid sequence of said light-chain variable region of said anti-IL-36R antibody, based on SEQ ID NO: 11.

37. The bispecific antibody according to any one of claims 22 to 36, wherein said ScFv of said anti-IL-36R antibody is linked to a region of said anti-IL-17A selected from regions in the group consisting of: a heavy-chain variable region, a heavy-chain constant region, or combinations thereof.

38. The bispecific antibody according to any one of claim 22 to 37, wherein said ScFv of said anti-IL-36R antibody is linked to a terminus of said heavy-chain constant region of said anti-IL-17A antibody.

39. The bispecific antibody according to any one of claims 22 to 38, wherein said ScFv of said IL-36R antibody is humanized.

40. The bispecific antibody according to any one of claims 22 to 39, wherein said ScFv of said anti-IL-36R antibody comprises an anti-IL-36R heavy-chain variable region and an anti-IL-36R light-chain variable region.

41. The bispecific antibody according to any one of claims 22 to 40, wherein said ScFv of said anti-IL-36R antibody further comprises a linker peptide located between said heavy-chain variable region of said anti-IL-36R antibody and said light-chain variable region of said anti-IL-36R antibody for linking said heavy-chain variable region to said light-chain variable region.

42. The bispecific antibody according to claim 41, wherein said linker peptide has an amino acid sequence of (G4S)n, with n being a positive integer.

43. The bispecific antibody according to any one of claims 22 to 42, wherein said anti-IL-17A antibody is humanized.

44. The bispecific antibody according to any one of claims 22 to 43, wherein said heavy-chain constant regions CH1, CH2 and CH3 and light-chain constant region CL of said anti-IL-17A antibody are both derived from human IgG1 or IgG4, which can be, for example, human IgG4.

45. The bispecific antibody according to any one of claims 1 to 44, wherein said bispecific antibody is a homodimer.

46. The bispecific antibody according to any one of claims 1 to 45, wherein said bispecific antibody is a tetravalent antibody.

47. The bispecific antibody according to any one of claims 1 to 46, wherein said bifunctional antibody has a structure from an N-terminus to a C-terminus as set forth in Formula I: 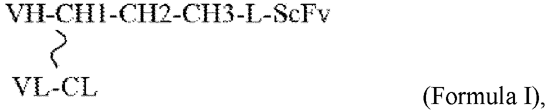 wherein
VH represents said heavy-chain variable region of said anti-IL-17A antibody;
VL represents said light-chain variable region of said anti-IL-17A antibody;
CHI, CH2 and CH3 represent said heavy-chain constant regions CH1, CH2 and CH3 of said anti-IL-17A antibody, respectively;
CL represents said light-chain constant region of said anti-IL-17A antibody;
ScFv represents said ScFv of said anti-IL-36R antibody;
L indicates a linker element;
"~" represents a disulfide bond;
"-" represents a peptide bond; and
wherein said bispecific antibody has an activity of simultaneously binding to said IL-17A and said IL-36R.

48. The bispecific antibody according to any one of claims 22 to 47, wherein said bispecific antibody is fused from said anti-IL-17A antibody and said ScFv of said anti-IL-36R antibody, and has two pairs of peptide chains symmetrical to each other, each pair of said peptide chains comprises a light chain L-chain and a heavy chain H-chain, and all of said peptide chains are linked by said disulfide bonds, wherein any pair of said peptide chains have structures of said H-chain and said L-chain from said N-terminus to said C-terminus as set forth in Formula I.

49. The bispecific antibody according to any one of claims 47 to 48, wherein said bispecific antibody is a homodimer of said peptide chains having said structures as set forth in Formula I.

50. The bispecific antibody according to any one of claims 47 to 49, wherein said linker element is a linker peptide.

51. The bispecific antibody according to any one of claims 47 to 50, wherein said linker element is a linker peptide, which has an amino acid sequence of (G4S)n, with n being a positive integer.

52. The bispecific antibody according to any one of claims 48 to 51, wherein said H-chain of said bispecific antibody has an amino acid sequence as set forth in any one of SEQ ID NO: 13 to 18; and said L-chain of said bispecific antibody has an amino acid sequence as set forth in SEQ ID NO: 19.

53. The bispecific antibody according to any one of claims 1 to 52, wherein said bispecific antibody further comprises a detectable label, a targeted marker, a drug, a toxin, a cytokine, a radionuclide, or an enzyme.

54. The bispecific antibody according to any one of claims 1 to 53, wherein said bispecific antibody is conjugated with a tumor-targeted marker conjugate.

55. A recombinant protein, comprising said bispecific antibody according to any one of claims 1 to 54, and optionally, a tag sequence assisting expression and/or purification.

56. The recombinant protein according to claim 55, wherein said tag sequence comprises a 6His tag.

57. 7. The recombinant protein according to any one of claims 55 to 56, comprising a fusion protein.

58. The recombinant protein according to any one of claims 55 to 57, wherein said recombinant protein is a monomer, dimer, or multimer.

59. A CAR construct, wherein an scFV segment of a monoclonal antibody antigen-binding region of said CAR construct is a binding region specifically binding to IL-36R, and said scFv has said heavy-chain variable region according to any one of claims 22 to 54 and said light-chain variable region according to any one of claims 22 to 54.

60. An immune cell expressing said CAR construct according to claim 59.

61. The immune cell according to claim 60, which is selected from the group consisting of: a NK cells, and a T cell.

62. The immune cell according to any one of claims 60 to 61, which is derived from human or a non-human mammal.

63. An antibody-drug conjugate, comprising an antibody moiety containing said bispecific antibody according to any one of claims 1 to 54, and a conjugated moiety conjugated to said antibody moiety, wherein said conjugated moiety is selected from the group consisting of: a detectable label, a drug, a toxin, a cytokine, a radionuclide, an enzyme, or combinations thereof.

64. The antibody-drug conjugate according to claim 63, wherein said antibody moiety is conjugated with said conjugated moiety via a chemical bond or linker.

65. A pharmaceutical composition, comprising an antibody moiety containing said bispecific antibody according to any one of claims 1 to 54, said immune cell according to any one of claims 60 to 62 and/or said antibody-drug conjugate according to any one of claims 63 to 64, and, a pharmaceutically acceptable carrier.

66. Use of said bispecific antibody according to any one of claims 1 to 54, said immune cell according to any one of claims 60 to 62, said pharmaceutical composition according to any one of claims 63 to 64 and/or said antibody-drug conjugate according to claim 65 as an active ingredient or active ingredients for (a) preparation of a detection reagent or kit; and/or (b) preparation of a drug for prevention and/or treatment of an IL-36-related disease.

67. Use of said bispecific antibody according to any one of claims 1 to 54, said immune cell according to any one of claims 60 to 62, said pharmaceutical composition according to any one of claims 63 to 64 and/or said antibody-drug conjugate according to claim 65 as an active ingredient or active ingredients for (a) preparation of a detection reagent or kit against IL-36 and/or IL-17; and/or (b) preparation of a drug for prevention and/or treatment of an IL-36- and/or IL-17-related disease.

68. The use according to claim 67, wherein said IL-36- and/or IL-17-related disease is an IL-36- and/or IL-17-mediated inflammatory disease.

69. The use according to any one of claims 67 to 68, wherein said IL-36- and/or IL-17-related disease is a disease induced by overstimulation or mutation of an IL-36 and/or IL-17 cytokine.

70. The use according to any one of claims 67 to 69, wherein said IL-36- and/or IL-17-related disease is selected from the group consisting of: inflammation, autoimmune diseases, or a combination thereof.

71. The use according to any one of claims 67 to 70, wherein said IL-36- and/or IL-17-related disease is selected from the group consisting of: psoriasis, scleroderma, a chronic kidney disease, an inflammatory bowel disease, psoriatic arthritis, ankylosing spondylitis, multiple sclerosis, inflammatory arthritis, asthma, allergy, or combinations thereof.

72. The use according to any one of claims 67 to 71, wherein said IL-36- and/or IL-17-related disease comprises psoriasis vulgaris, erythrodermic psoriasis, arthritic psoriasis, generalized pustular psoriasis (GPP), and palmoplantar pustular psoriasis (PPP).

73. The use according to any one of claims 67 to 72, wherein said drug is for use in blocking of binding of IL-36α, IL-36β or IL-36γ to IL-36R.

74. The use according to any one of claims 67 to 73, wherein said drug is also for use in blocking of an interaction between IL-17A and IL-17R.

75. The use according to any one of claims 67 to 74, wherein said drug is for use in blocking of cytokine secretion mediated by IL-36α, IL-36β or IL-36γ.

76. The use according to any one of claims 67 to 75, wherein said drug is also for use in blocking of cytokine secretion mediated by IL-17A.

77. The use according to any one of claims 67 to 76, wherein said detection reagent or kit is for use in diagnosis of an IL-36- and/or IL-17-related disease.

78. The use according to any one of claims 67 to 77, wherein said detection reagent or kit is for use in detection of an IL-36R and/or IL-17A protein in a sample.

79. The use according to any one of claims 67 to 78, wherein said detection reagent is a test tablet.

80. A polynucleotide encoding said bispecific antibody according to any one of claims 1 to 54, said recombinant protein according to any one of claims 60 to 62 and/or said CAR construct according claim 59.

81. A vector comprising said polynucleotide according to claim 80.

82. A host cell comprising said vector according to claim 81 or having said polynucleotide according to claim 80 incorporated in a genome.

83. A test plate, comprising: a substrate (support plate) and a test strip, wherein said test strip comprises said bispecific antibody according to any one of claims 1 to 54, and/or said antibody-drug conjugate according to any one of claims 63 to 64.
